# EUROPÄISCHE PATENTSCHRIFT

(11) **EP 1 076 646 B1**
(45) Veröffentlichungstag und Bekanntmachung des Hinweises auf die Patenterteilung: **04.08.2004**
(21) Anmeldenummer: 99934470.8
(22) Anmeldetag: 10.05.1999
(51) Int. Cl.: C07D 207/12, C07D 209/52, C07D 291/04, C07C 381/10, C07F 7/10

(54) **VERFAHREN ZUR STEREOCHEMISCH KONTROLLIERTEN HERSTELLUNG ISOMERENREINER HOCHSUBSTITUIERTER AZACYCLISCHER VERBINDUNGEN**
METHOD FOR STEREOCHEMICALLY CONTROLLED PRODUCTION OF ISOMERICALLY PURE HIGHLY SUBSTITUTED AZACYCLIC COMPOUNDS
PROCEDE POUR LA PRODUCTION STEREOCHIMIQUEMENT CONTROLEE DE COMPOSES AZACYCLIQUES ISOMERIQUEMENT PURS HAUTEMENT SUBSTITUES

(30) Priorität: 13.05.1998 DE 19821418
(43) Veröffentlichungstag der Anmeldung: 21.02.2001
(73) Patentinhaber: Solvay Pharmaceuticals GmbH, 30173 Hannover (DE)
(72) Erfinder: REGGELIN, Michael, D-65589 Hadamar (DE); HEINRICH, Timo, D-63517 Rodenbach (DE); JUNKER, Bernd, D-65812 Bad Soden (DE); ANTEL, Jochen, D-31848 Bad Münder (DE); PREUSCHOFF, Ulf, D-29125 Uelzen (DE)
(74) Vertreter: Gosmann, Martin, Dr.
(86) Internationale Anmeldenummer: PCT/DE1999/001417
(87) Internationale Veröffentlichungsnummer: WO 1999/058500

(56) Entgegenhaltungen:
- EP-A- 0 394 991
- EP-A- 0 558 443
- COMINS, DANIEL L. ET AL: "Regio- and stereoselective addition of nucleophiles to 1-phenoxycarbonyl-2,3-dihydropyridinium salts" HETEROCYCLES (1994), 37(2), 1121-40 , XP002119392
- BEAK, PETER ET AL: ".alpha.-Lithioamine synthetic equivalents: syntheses of diastereoisomers from Boc derivatives of cyclic amines" J. ORG. CHEM. (1993), 58(5), 1109-17 , XP002119393
- TORII, SIGERU ET AL: "Facile access to 6-methoxy-1,2,3,6-tetrahydro- and 4-hydroxy-1,2,3,4- tetrahydropyridines by electrochemical haloalkoxylation- dehydrohalogenation sequence as a key operation" SYNTHESIS (1987), (3), 242-5 , XP002119394
- CHEMICAL ABSTRACTS, vol. 81, no. 23, 9. Dezember 1974 (1974-12-09) Columbus, Ohio, US; abstract no. 151295, MELESHINA, A. M. ET AL: "Calculation of the effect of substituents on the vibrational spectra of.alpha.- and.beta.-isomers of 2,6-dimethyl-4-hydroxy-1,2,3,4- tetrahydroquinolines" XP002119399 & ZH. PRIKL. SPEKTROSK. (1974), 21(2), 286-90 ,
- SCHINDLER, OTHMAR ET AL: "Stereochemistry of the intermediates (aldol bases) of the Doebner-von Miller quinoline synthesis" HELV. CHIM. ACTA (1970), 53(4), 776-9 , XP002119395
- REGGELIN, MICHAEL ET AL: "Metalated 2-Alkenylsulfoximines: Efficient Solutions for Asymmetric d3-Synthons" J. AM. CHEM. SOC. (1996), 118(20), 4765-77 , XP002119396 in der Anmeldung erwähnt
- REGGELIN, MICHAEL ET AL: "One-pot synthesis of (S)-4-isopropyl-2-p-toluene-4,5-dihydro- [1,2.lambda.6,3]oxathiazole 2-oxides: efficient precursors of optically active sulfoximines" TETRAHEDRON LETT. (1992), 33(46), 6959-62 , XP002119397
- REGGELIN, MICHAEL ET AL: "Metalated 2-Alkenylsulfoximides in asymmetric synthesis: diastereoselective preparation of highly substituted pyrrolidine derivatives" ANGEW. CHEM., INT. ED. (1998), 37(20), 2883-2886 , XP002119398

## Beschreibung

Die vorliegende Erfindung betrifft ein neues Verfahren zur stereochemisch kontrollierten Herstellung neuer und bekannter hochsubstituierter azacyclischer Verbindungen sowie neue Zwischenprodukte dieses Verfahrens. Weiterhin betrifft die Erfindung neue hochsubstituierte azacyclische Verbindungen, welche isomerenrein aufgebaut werden können und welche für zahlreiche Anwendungsgebiete nützliche Eigenschaften aufweisen.

Hochsubstituierte Stereoisomere von azacyclischen Verbindungen, insbesondere hochsubstituierte Derivate von Pyrrolidinen oder Piperidinen, stellen für zahlreiche Anwendungen nützliche Ausgangsstoffe dar und finden beispielsweise Verwendung als Bestandteile von chiralen Katalysatoren in der asymmetrischen Synthese (siehe z. B. Kobayashi et al., Chemistry Letters (= Chem. Lett.) (1991) 1341-1344), als Bestandteile biologisch aktiver Alkaloide (siehe z. B. Williams et al., Journal of Organic Chemistry (= JOC) 57 (1992) 6527-6532 und darin zitierte Referenzen; Jäger et al., Angewandte Chemie 102 (1990) 1180-1182) sowie als Bestandteile pharmakologisch interessanter Verbindungen (siehe z. B. Laschat et al., Synthesis 4 (1997) 475-479). Weiterhin weisen beispielsweise nach dem erfindungsgemäßen Verfahren herstellbare oder strukturell eng verwandte Decahydrochinoline und Pyrrolidine interessante physiologische Wirkungen auf (siehe z. B. Kuzmitskii et al., Vestsi Akad. Navuk BSSR, Ser. Khim. Navuk 3 (1979) 82-85/Chemical Abstracts Nr. 91:117158c; Lash et al., Journal of Heterocyclic Chemistry 28 (1991) 1671-1676). Auch die Verwendung einiger vorstehend angegebener Pyrrolidine zur Herstellung von Porphyrin-Ringsystemen wird dort diskutiert. Aus den angegebenen Literaturstellen sind z. T. auch Verfahren zur Herstellung derartiger azacyclischer Verbindungen bekannt. Bestimmte Enantiomere dieser Verbindungen können nach den dort angegebenen Methoden üblicherweise mittels konventioneller Racemattrennung erhalten werden. Es sind aber auch nicht-erfindungsgemäße Herstellungsverfahren angegeben, nach denen ausgewählte Einzelverbindungen substituierter Azacyclen isomerenrein hergestellt werden können. Ein allgemeines Verfahren zur stereokontrollierten Synthese isomerenreiner hochsubstituierter Azacyclen ist aus den vorstehend angegebenen Literaturstellen nicht bekannt.

Weiterhin ist bereits die stereokontrollierte Synthese einiger Tetrahydrofuran-Derivate durch Umsetzung von 2-Alkenyl-Sulfoximiden mit 2-tert.-Butyldimethylsilyloxy-propanal (= TBS-Lactaldehyd) und nachfolgende Fluorid-induzierte Cyclisierung bekannt (siehe Reggelin et al., JACS 118 (1996) 4765-4777; Reggelin et al., Liebigs Annalen der Chemie/RECUEIL (1997) 1881-1886). Hochsubstituierte azacyclische Verbindungen können nach dem dort beschriebenen Verfahren jedoch nicht hergestellt werden.

Aus der Veröffentlichung im Internet unter der Adresse "www.iucr.ac.uk" von M. Bolte, Acta Crystallographica Section C, electronically published paper QA0017 [=(IUCr) Acta C Paper QA 0017] ist bereits die Verbindung (2S,3S,4S,5S)-(N-tert.-Butyloxycarbonyl)-2-benzyl-4,5-dimethyl-3-hydroxypyrrolidin bekannt. Die Herstellung dieser Verbindung wird in der angegebenen Veröffentlichung nicht beschrieben.

Aufgabe der vorliegenden Erfindung war es, ein Verfahren zur stereochemisch kontrollierten Herstellung neuer und bekannter hochsubstituierter azacyclischer Verbindungen zur Verfügung zu stellen, womit auch die Art und Anzahl der Substituenten in diesen Verbindungen breit variiert werden kann und welche isomerenrein aufgebaut werden können. Weiterhin war es Aufgabe der Erfindung, neue, insbesondere isomerenreine hochsubstituierte azacyclische Verbindungen für zahlreiche Anwendungsbereiche zur Verfügung zu stellen.

Es wurde nun überraschend gefunden, daß hochsubstituierte azacyclische Verbindungen, worin Art und Anzahl der Substituenten breit variierbar sind, in guter Ausbeute insbesondere isomerenrein aufgebaut werden können, wenn man nach einem erfindungsgemäßen Verfahren metallierte 2-Alkenylsulfoximid-Verbindungen mit N-geschützten α- oder β-Aminoaldehyden umsetzt, welche in α- und/oder in β-Stellung das in der Beschreibung angegebene Substitutionsmuster aufweisen können.

Gegenstand der Erfindung ist somit ein Verfahren zur stereochemisch kontrollierten Herstellung von Verbindungen der allgemeinen Formel I, 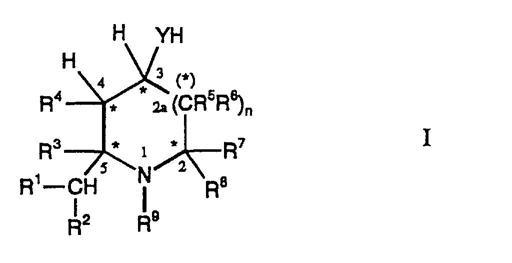 worin
- n: 0 oder 1 bedeutet,
- R¹: Wasserstoff, C₁-C₆-Alkyl oder gegebenenfalls im Phenylring ein- oder mehrfach durch niederes Alkyl, niederes Haloalkyl, niederes Alkoxy oder niederes Haloalkoxy substituiertes Phenyl-C₁-C₆-alkyl bedeutet und
- R²: Wasserstoff bedeutet, oder
- R¹ und R²: gemeinsam eine doppelt gebundene Methylengruppe bedeuten, welche durch C₁-C₅-Alkyl oder gegebenenfalls im Phenylring ein- oder mehrfach durch niederes Alkyl, niederes Haloalkyl, niederes Alkoxy oder niederes Halo-alkoxy substituiertes Phenyl-C₁-C₅-alkyl substituiert sein kann,
- R³: Wasserstoff bedeutet und
- R⁴: Wasserstoff, niederes Alkyl oder gegebenenfalls im Phenylring ein- oder mehrfach durch niederes Alkyl, niederes Haloalkyl, niederes Alkoxy oder niederes Haloalkoxy substituiertes Phenylniederalkyl bedeutet, oder
- R³ und R⁴: auch gemeinsam eine C₂-Alkylenkette oder eine gegebenenfalls 1 bis 3 Doppelbindungen enthaltende C₃-C₆-Alkylenkette bedeutet, welche durch gegebenenfalls ein- oder zweifach durch niederes Alkyl substituiertes C₁-C₂-Alkylen überbrückt sein kann,
- R⁵: Wasserstoff, niederes Alkyl, Hydroxy, niederes Alkoxy oder jeweils im Phenylring gegebenenfalls ein- oder mehrfach durch niederes Alkyl, niederes Haloalkyl, niederes Alkoxy oder niederes Haloalkoxy substituiertes Phenylniederalkyl oder Phenylniederalkoxy bedeutet und
- R⁶: Wasserstoff bedeutet und
- R⁷: Wasserstoff bedeutet und
- R⁸: Wasserstoff, Cyano, gegebenenfalls verestertes Carboxy, gegebenenfalls am Stickstoff ein- oder zweifach substituiertes Carbonylamino, ein gegebenenfalls ein- oder mehrfach ungesättigtes mono- oder bicyclisches Ringsystem mit 3 bis 10 Ringkohlenstoffatomen, dessen Ringkohlenstoffatome ein- oder mehrfach durch Stickstoff, Sauerstoff und/oder Schwefel ersetzt sein können und welches Ringsystem ein- oder mehrfach durch niederes Alkyl, niederes Haloalkyl, niederes Alkoxy, Hydroxy, Halogen oder durch eine niedere Alkylenkette, welche an zwei an benachbarte Kohlenstoffatome des Ringsystems gebundene Sauerstoffatome gebunden ist, substituiert sein kann, bedeutet, oder
auch für gegebenenfalls ein- oder mehrere Doppelbindungen enthaltendes geradkettiges oder verzweigtes C₁-C₁₂-Alkyl stehen kann, welches ein- oder mehrfach durch Halogen, Hydroxy, niederes Alkoxy, gegebenenfalls verestertes Carboxy, Cyano, Mercapto, niederes Alkylthio, Amino, niederes Alkylamino, gegebenenfalls am Stickstoff ein- oder zweifach substituiertes Carbonylamino, ein gegebenenfalls ein- oder mehrfach ungesättigtes mono- oder bicyclisches Ringsystem mit 3 bis 10 Ringkohlenstoffatomen, dessen Ringkohlenstoffatome ein- oder mehrfach durch Stickstoff, Sauerstoff und/oder Schwefel ersetzt sein können und welches Ringsystem ein- oder mehrfach durch niederes Alkyl, niederes Haloalkyl, niederes Alkoxy, Hydroxy, Halogen oder durch eine niedere Alkylenkette, welche an zwei an benachbarte Kohlenstoffatome des Ringsystems gebundene Sauerstoffatome gebunden ist, substituiert sein kann, oder
- R⁵ und R⁸: auch gemeinsam mit den Kohlenstoffatomen, an welche sie gebunden sind, ein gegebenenfalls 1 bis 3 Doppelbindungen enthaltendes mono- oder bicyclisches Ringsystem mit 5 bis 10 Ringkohlenstoffatomen bilden können, dessen nicht die Substituenten R⁵ oder R⁸ tragende Kohlenstoffatome ein- oder mehrfach durch Schwefel, Sauerstoff und/oder Stickstoff ersetzt sein können, und welches gegebenenfalls ein- oder mehrfach durch niederes Alkyl, niederes Haloalkyl, niederes Alkoxy, niederes Haloalkoxy, Hydroxy, Halogen oder durch eine niedere Alkylenkette, welche an zwei an benachbarte Kohlenstoffatome des Ringsystems gebundene Sauerstoffatome gebunden ist, substituiert sein kann, oder
- R⁶ und R⁷: auch gemeinsam eine Bindung bilden können und
- R⁵ und R⁸: gemeinsam mit den Kohlenstoffatomen, an welche sie gebunden sind, ein aromatisches C₆-Ringsystem bilden können, welches mit-2 bis 4 weiteren Kohlenstoffatomen zu einem insgesamt 8 bis 10 Ringkohlenstoffatome enthaltenden bicyclischen Ringsystem mit insgesamt 3 bis 5 Doppelbindungen anelliert sein kann, wobei die nicht die Substituenten R⁵ und R⁸ tragenden Kohlenstoffatome dieses C₆- bis C₁₀-Ringsystems ein- oder mehrfach durch Schwefel, Sauerstoff und/oder Stickstoff ersetzt sein können und wobei dieses C₆- bis C₁₀-Ringsystem gegebenenfalls ein- oder mehrfach durch niederes Alkyl, niederes Haloalkyl, niederes Alkoxy, niederes Haloalkoxy, Hydroxy, Halogen oder durch eine niedere Alkylenkette, welche an zwei an benachbarte Kohlenstoffatome des Ringsystems gebundene Sauerstoffatome gebunden ist, substituiert sein kann,
- R⁹: Wasserstoff, niederes Alkyl, gegebenenfalls im Phenylring ein- oder mehrfach durch niederes Alkyl, niederes Haloalkyl, niederes Alkoxy oder niederes Haloalkoxy substituiertes Phenylniederalkyl oder eine Aminoschutzgruppe bedeutet, oder
- R⁸ und R⁹: auch gemeinsam eine C₃-C₄-Alkylenkette bilden können und
- Y: Sauerstoff oder NH bedeutet,
und deren Säureadditionssalzen, wobei gegebenenfalls vorhandene reaktive Gruppen in Verbindungen der Formel I durch geeignete Schutzgruppen blockiert sein können, dadurch gekennzeichnet, daß man
a) eine Verbindung der allgemeinen Formel II, 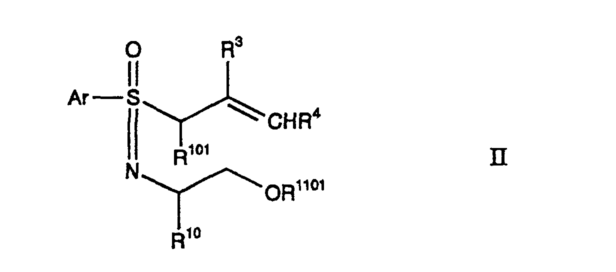 worin R³ und R⁴ obige Bedeutungen besitzen, R¹⁰¹ die vorstehend für R¹ angegebene Bedeutung mit Ausnahme einer gegebenenfalls substituierten Methylengruppe besitzt, Ar für gegebenenfalls ein- oder mehrfach durch niederes Alkyl substituiertes Phenyl steht, R¹⁰ niederes Alkyl oder gegebenenfalls im Phenylring einfach durch niederes Alkyl oder durch mit einer geeigneten Schutzgruppe geschütztes Hydroxy substituiertes Phenyl oder gegebenenfalls im Phenylring einfach durch niederes Alkyl substituiertes Phenylniederalkyl bedeutet und R¹¹⁰¹ für eine Silyl-Schutzgruppe steht, nacheinander mit einer zu deren Deprotonierung geeigneten Base, einem metallorganischen Reagenz der allgemeinen Formel VII,

   **XM**^{**2**}**(OR**^{**12**}**)**_{**3**} **VII**

   worin X für Halogen steht, M² ein vierwertiges Übergangsmetall bedeutet und R¹² für niederes Alkyl, Phenyl oder Phenylniederalkyl steht, und einem Stereoisomer einer Verbindung der allgemeinen Formel VIII, 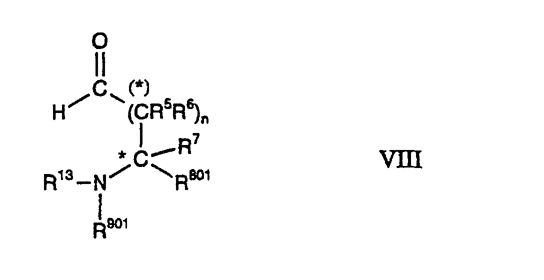 worin R⁵, R⁶, R⁷ und n die obigen Bedeutungen besitzen, R⁸⁰¹ die Bedeutung von R⁸ besitzt, wobei allfällige reaktive Gruppen nötigenfalls durch basenstabile Schutzgruppen blockiert sind, R⁹⁰¹ für Wasserstoff oder gemeinsam mit R⁸⁰¹ für eine C₃-C₄-Alkylenkette steht und R¹³ eine Amino-Schutzgruppe bedeutet, welche bei ihrer Abspaltung ein Stickstoff-Nucleophil hinterläßt, umsetzt zu einem Stereoisomer einer Verbindung der allgemeinen Formel IX, 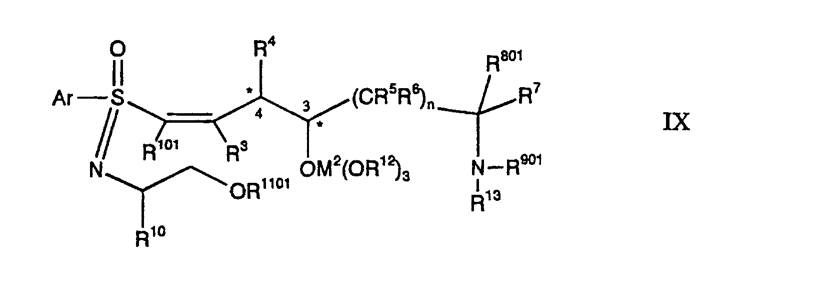 worin R¹⁰¹, R³, R⁴, R⁵, R⁶, R⁷, R⁸⁰¹, R⁹⁰¹, R¹⁰, R¹¹⁰¹, R¹², R¹³, n, Ar und M² obige Bedeutungen besitzen,
b) die erhaltene Verbindung der Formel IX durch Behandlung mit einem zur Entfernung der Gruppe R¹³ geeigneten Reagenz überführt in eine Verbindung der allgemeinen Formel Xa, 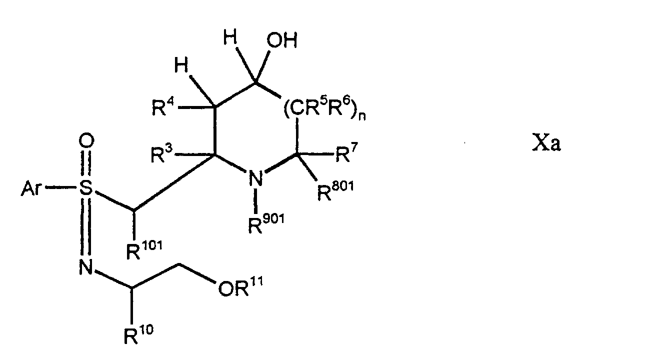 worin R¹⁰¹, R³, R⁴, R⁵, R⁶, R⁷, R⁸⁰¹, R⁹⁰¹, R¹⁰ , n und Ar obige Bedeutungen besitzen und R¹¹ für Wasserstoff oder eine Silylschutzgruppe steht und, sofern R⁹⁰¹ für Wasserstoff steht, das Stickstoffatom im cyclischen Grundgerüst der entstandenen Verbindung der Formel Xa mit einer basenstabilen Schutzgruppe blockiert und eine gegebenenfalls noch vorhandene Silylschutzgruppe R¹¹ abspaltet, und
c) zur Herstellung einer Verbindung der allgemeinen Formel Ia 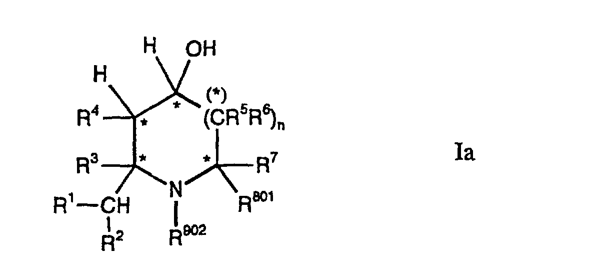 worin R¹, R², R³, R⁴, R⁵, R⁶, R⁷, R⁸⁰¹ und n obige Bedeutungen besitzen und R⁹⁰² für eine basenstabile Schutzgruppe oder gemeinsam mit R⁸⁰¹ für eine C₃-C₄-Alkylenkette steht,
   ca) eine erhaltene Verbindung der Formel Xa oder eine durch Abspaltung der Silylschutzgruppe R¹¹ entstandene Verbindung mit einem zur reduktiven Spaltung der Sulfonimidoyl-Alkyl-Bindung geeigneten Reagenz umsetzt, um eine Verbindung der allgemeinen Formel Ib, 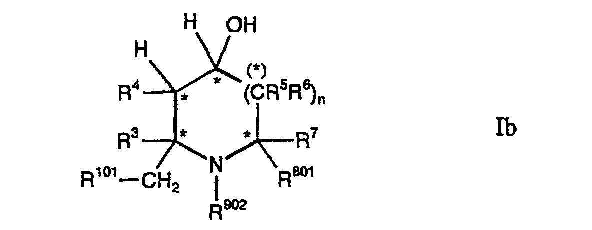 worin R¹⁰¹, R³, R⁴, R⁵, R⁶, R⁷, R⁸⁰¹, R⁹⁰² und n obige Bedeutungen besitzen, zu erhalten, oder
   cb) in einer erhaltenen Verbindung der Formel Xa, worin R¹⁰¹ nicht für Wasserstoff steht, die Sulfonimidoyl-Alkyl-Bindung nach elektrophiler Aktivierung der Sulfonimidoyl-Einheit unter den Bedingungen einer baseninduzierten Eliminierung spaltet, um eine Verbindung der allgemeinen Formel Ic, 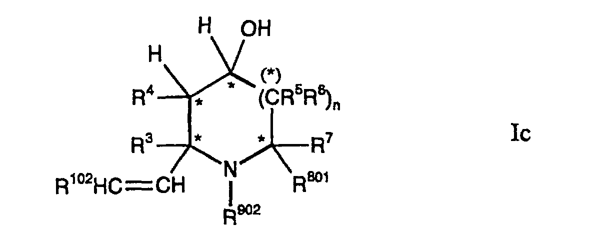 worin R³, R⁴, R⁵, R⁶, R⁷, R⁸⁰¹, R⁹⁰² und n obige Bedeutungen besitzen und R¹⁰² für C₁-C₅-Alkyl steht oder für gegebenenfalls im Phenylring ein- oder mehrfach durch niederes Alkyl, niederes Haloalkyl, niederes Alkoxy oder niederes Haloalkoxy substituiertes Phenylniederalkyl steht, dessen niedere Alkylenkette 1 bis 5 Kohlenstoffatome enthalten kann, zu erhalten,
   und eine erhaltene Verbindung der Formel Ia gewünschtenfalls einmal oder mehrmals durch Umsetzung, jeweils unter Inversion der Konfiguration am Ringkohlenstoffatom in 3-Position der Verbindungen der Formel Ia, mit einem zur Wiedererzeugung einer OH-Gruppe oder zu Erzeugung einer NH₂-Gruppe in der 3-Position geeigneten nucleophilen Reagenz umsetzt und/oder gewünschtenfalls allfällige Schutzgruppen in Verbindungen der Formel Ia wieder abspaltet und gewünschtenfalls die gegebenenfalls freigesetzte NH-Gruppe in 1-Position des cyclischen Grundgerüstes mit einem zur N-Alkylierung oder einem zur Amidbildung befähigten Reagenz umsetzt oder mit einer Aminoschutzgruppe blockiert, um Verbindungen der Formel I zu erhalten und freie Verbindungen der Formel I gewünschtenfalls zu Säureadditionssalzen umsetzt, oder Säureadditionssalze von Verbindungen der Formel I zu freien Verbindungen umsetzt. Weiterhin sind Gegenstand der Erfindung neue azacyclische Verbindungen.

Sofern in Verbindungen der Formel I oder in anderen im Rahmen der vorliegenden Erfindung beschriebenen Verbindungen Substituenten niederes Alkyl bedeuten oder enthalten, kann dieses verzweigt oder unverzweigt sein und üblicherweise 1 bis 4 Kohlenstoffatome enthalten.

Sofern in den Definitionen der Substituenten von Verbindungen der Formel I oder der Formel X Substituentenbestandteile, beispielsweise an Phenylringe gebundene Reste, ein- oder mehrfach enthalten sein können, können diese üblicherweise ein- bis dreifach enthalten sein. Sofern in Verbindungen der vorliegenden Erfindung ein oder mehrere Kohlenstoffatome durch Heteroatome wie Sauerstoff, Schwefel oder Stickstoff ersetzt sein können, können üblicherweise ein bis drei Kohlenstoffatome durch Heteroatome ersetzt sein. Vorzugsweise kann ein Kohlenstoffatom durch ein Heteroatom ersetzt sein. Sofern Substituenten eine oder mehrere Doppelbindungen enthalten können, können cyclische Substituenten, je nach Ringgröße, üblicherweise 1 - 4 Doppelbindungen enthalten und können bevorzugt aromatische Systeme bilden. Aliphatische Substituenten können je nach Kettenlänge beispielsweise 1 bis 3 Doppelbindungen enthalten.

Vorzugsweise können Verbindungen der Formel Ia hergestellt werden, worin die Substituenten R¹ und R² jeweils für Wasserstoff stehen. Besonders bevorzugt können Verbindungn der allgemeinen Formel Ib hergestellt werden, insbesondere dann, wenn der Substituent R¹⁰¹ Wasserstoff bedeutet.

Der Substituent R³ kann vorzugsweise für Wasserstoff stehen oder kann gemeinsam mit R⁴ eine gegebenenfalls überbrückte C₃-C₆-Alkylenkette bilden. Bevorzugt können solche Verbindungen der Formel I isomerenrein hergestellt werden, worin R⁴ nicht Wasserstoff, sondern beispielsweise niederes Alkyl, bedeutet. Sofern R⁴ eine andere Bedeutung als Wasserstoff besitzt, verläuft die Ringschlußreaktion zu Verbindungen der Formel Xa in Verfahrensschritt b) mit einer besonders hohen Selektivität und die aus den Verbindungen der Formel Xa erhaltenen Verbindungen der Formel Ia und der Formel I können mit einem besonders geringen Anteil an Nebenprodukten erhalten werden. Sofern R³ und R⁴ gemeinsam für eine gegebenenfalls überbrückte C₃-C₆-Alkylenkette stehen, kann die Alkylenkette vorzugsweise 3 bis 4 Kohlenstoffatome enthalten. Sofern die Alkylenkette überbrückt ist, kann die überbrückende Kette vorzugsweise 1 Kohlenstoffatom besitzen, welches vorzugsweise durch Diniederalkyl substituiert sein kann. Insbesondere können R³ und R⁴ gemeinsam mit den Kohlenstoffatomen, woran sie gebunden sind, das 7,7-Dimethylbicyclo[3.1.1]heptan-System bilden.

Sofern der Substituent R⁸ gegebenenfalls verestertes Carboxy bedeutet oder enthält, kann die Carboxylgruppe mit üblichen, nicht sterisch gehinderten Alkoholen verestert sein, beispielsweise mit gegebenenfalls ein- oder mehrere Doppelbindungen enthaltenden cycloaliphatischen oder geradkettigen oder verzweigten aliphatischen C₁-C₆-Alkoholen, welche gegebenenfalls ein- oder mehrfach durch Halogen oder niederes Alkoxy substituiert sein können, oder auch mit gegebenenfalls im Phenylring ein- oder mehrfach durch niederes Alkyl, niederes Haloalkyl, niederes Alkoxy oder niederes Haloalkoxy substituierten Phenylniederalkylalkoholen. Sofern R⁸ gegebenenfalls am Stickstoff ein- oder zweifach substituiertes Carbonylamino bedeutet oder enthält, kann die darin enthaltene Aminogruppe beispielsweise einfach substituiert sein durch C₃-C₈-Cyloalkylniederalkanoyl oder geradkettiges oder verzweigtes aliphatisches C₁-C₆-Alkanoyl, welche gegebenenfalls jeweils ein- oder mehrfach durch Halogen oder niederes Alkoxy substituiert sein können, oder die Aminogruppe kann einfach substituiert sein durch gegebenenfalls im Phenylring ein- oder mehrfach durch niederes Alkyl, niederes Haloalkyl, niederes Alkoxy oder niederes Haloalkoxy substituiertes Phenylniederalkanoyl oder die Aminogruppe kann beispielsweise auch ein- oder zweifach substituiert sein durch C₃-C₈-Cycloalkylniederalkyl oder geradkettiges oder verzweigtes aliphatisches C₁-C₆-Alkyl, welche gegebenenfalls jeweils ein- oder mehrfach durch Halogen oder niederes Alkoxy substituiert sein können, gegebenenfalls im Phenylring ein- oder mehrfach durch niederes Alkyl, niederes Haloalkyl, niederes Alkoxy oder niederes Haloalkoxy substituiertes Phenylniederalkyl oder die Aminogruppe kann beispielsweise mit einer geeigneten Aminoschutzgruppe geschützt sein. Sofern R⁸ ein gegebenenfalls substituiertes mono- oder bicyclisches Ringsystem mit 3 bis 10 Ringkohlenstoffatomen bedeutet oder enthält, kann dieses beispielsweise für Cyclopropyl, Cyclopentyl, Cyclohexyl, Phenyl, p-Bromphenyl oder 3-Indolyl stehen.

Beispiele für erfindungsgemäße Verbindungen der Formel I, Ia, Ib und/oder Ic, welche sich nach dem erfindungsgemäßen Verfahren problemlos herstellen lassen, weisen als Substituenten R⁸ bzw. R⁸⁰¹ Wasserstoff, Niederalkyl, Phenyl, Niederalkylphenyl oder Niederalkyloxyniederalkyl auf oder enthalten beispielsweise auch einen aus R⁸, bzw. R⁸⁰¹, R⁵, R⁶ und R⁷ gebildeten anellierten aromatischen 6-Ring. Ebenso lassen sich Verbindungen der Formeln I, Ia, Ib, und/oder Ic problemlos herstellen, worin R⁸⁰¹ gemeinsam mit R⁹⁰¹ eine C₃-C₄-Alkylenkette bildet.

Geeignete Schutzgruppen, welche in den im Rahmen der vorliegenden Erfindung angegebenen Verbindungen verwendet werden können, sind beispielsweise bekannt aus McOmie, "Protective Groups in Organic Chemistry", Plenum Press oder aus Green, Wuts, "Protective Groups in Organic Synthesis", Wiley Interscience Publication.

Die Deprotonierung von Verbindungen der Formel II mit geeigneten Basen und die Umsetzung der deprotonierten Verbindungen der Formel II mit metallorganischen Reagenzien der Formel VII und anschließend mit den Aminoaldehyden der Formel VIII zu den Verbindungen der Formel IX in Verfahrensschritt a) kann in einem unter den Reaktionsbedingungen inerten polaren oder schwach polaren aprotischen Lösungsmittel, beispielsweise in cyclischen oder offenkettigen Niederalkylethern wie Diethylether (= Ether) oder Tetrahydrofuran (= THF), in niedermolekularen Polyethylenglykolethern wie Diethylendimethylether (= Diglyme) oder in substituierten Benzolen wie Toluol oder Xylol durchgeführt werden. Vorzugsweise können schwach polare Lösungsmittel wie substituierte Benzole, insbesondere Toluol, verwendet werden. Sofern Toluol als Lösungsmittel verwendet wird, werden besonders gute Ausbeuten der Produkte der Formel IX bzw. der daraus erhaltenen Produkte der Formel Xa erhalten. Vorteilhaft kann die Reaktion als Eintopf-Reaktion ausgeführt werden, indem man ein vorzugsweise isomerenreines 2-Alkenylsulfoximid der Formel II in einem vorstehend genannten geeigneten Lösungsmittel bei tiefer Temperatur, beispielsweise zwischen -100 °C und -50 °C, vorzugsweise bei -78 °C, etwa 5 bis 30 Minuten lang mit einer geeigneten Base deprotoniert, die deprotonierte Form der Verbindung der Formel II bei leicht erhöhter Temperatur, beispielsweise zwischen -20 °C und 10 °C, vorzugsweise bei 0 °C, mit einem metallorganischen Reagenz der Formel VII transmetalliert und anschließend wieder bei tiefer Temperatur, beispielsweise zwischen -100 °C und -50 °C, vorzugsweise bei -78 °C, das erhaltene Zwischenprodukt mit einem N-geschützten Aminoaldehyd der Formel VIII umsetzt. Als Basen zur Deprotonierung von Verbindungen der Formel II eignen sich vorzugsweise lithiierte Niederalkylverbindungen wie n-Butyl-lithium. Üblicherweise kann die Base in einem geringen Überschuß, beispielsweise im Molverhältnis von etwa 1:1,05 bis etwa 1:1,20, bezogen auf die Menge der eingesetzten Verbindung der Formel II, verwendet werden. In metallorganischen Reagenzien der Formel VII kann X für Halogen, vorzugsweise für Chlor stehen. Als vierwertiges Übergangsmetall M² kann beispielsweise Zirkon, vorzugsweise aber Titan verwendet werden. Als Substituenten R¹² eignen sich beispielsweise verzweigte und unverzweigte Niederalkylgruppen, vorzugsweise Isopropyl. Besonders bevorzugt kann als Verbindung der Formel VII Chlortris(isopropoxy)titan verwendet werden. Das metallorganische Reagenz wird vorteilhaft in einem geringen Überschuß, beispielsweise im Molverhältnis von etwa 1,1:1 bis 1,3:1, bezogen auf die eingesetzte Menge der Verbindung der Formel II, verwendet.

Die Verbindungen der Formel VIII stellen geschützte chirale α- oder β- Aminoaldehyde dar und können vorzugsweise isomerenrein eingesetzt werden. Als bei Ihrer Abspaltung ein nucleophiles Stickstoffatom in Verbindungen der Formel VIII erzeugende Schutzgruppen R¹³ eignen sich vorzugsweise basenlabile Schutzgruppen. Besonders bevorzugt kann die Fluoren-9-yl-methyloxycarbonyl-Schutzgruppe (= FMOC) als Gruppe R¹³ verwendet werden. Die Abspaltung der Schutzgruppe R¹³ und die Ringschlußreaktion können vorzugsweise in einem einzigen Reaktionsschritt erfolgen, sofern FMOC als Schutzgruppe eingesetzt wird.

In den Ausgangsverbindungen der Formel VIII besitzt der Substituent R⁸⁰¹ die für R⁸ angegebene Bedeutung, wobei jedoch allenfalls im Substituenten R⁸ enthaltene reaktive Gruppen, beispielsweise Hydroxy, Amino, Mercapto oder Carboxy, jeweils durch an sich bekannte basenstabile Schutzgruppen, beispielsweise gegen nicht-nucleophile oder schwach-nucleophile Basen wie Pyridin stabile Schutzgruppen, blockiert sind, um unerwünschte Nebenreaktionen zu vermeiden. Isomerenreine Aminoaldehyde der Formel VIII sind bekannt oder können auf an sich bekannte Weise aus bekannten Verbindungen hergestellt werden. So können beispielsweise die Aldehyde der Formel VIII durch an sich bekannte schonende Oxidationsverfahren aus den zu den Aldehyden korrespondierenden primären Alkoholen erhalten werden. Als-schonende Oxidationsverfahren eignen sich solche Verfahren, welche keine Racemisierung der Chiralitätszentren in Verbindungen der Formel VIII verursachen, beispielsweise die Oxidation mit aktiviertem Oxalylchlorid (= Swern-Oxidation) oder auch die Oxidation mit 1,1,1-Triacetoxy-1,1-dihydro-1,2-benziodoxol-3(1H)-on (= Periodinan; Dess-Martin-Oxidation, siehe z. B. J. C. Martin et al. JACS 113 (1991) 7277-7287; D. B. Dess, J. C. Martin, Journal of Organic Chemistry 48 (1983) 4155 - 4156). Sofern die Oxidation nach der vorstehend angegebenen Dess-Martin Methode erfolgt, kann ein Aminoaldehyd der Formel VIII nach einem in der vorstehenden Literatur angegebenen Verfahren oder einem hierzu analogen Verfahren hergestellt werden. Beispielsweise kann ein als Vorläufer für einen Aldehyd der Formel VIII in Frage kommender primärer Alkohol in einem dipolar-aprotischen Lösungsmittel, beispielsweise in einem halogenierten niederen Alkan wie Dichlormethan, mit einem geringen Überschuß des Triacetoxy-Periodinans, beispielsweise im Molverhältnis von etwa 1,2:1 bis etwa 1,4:1, bezogen auf die eingesetzte Verbindung der Formel VIII, umgesetzt werden. Die Reaktion kann bei Temperaturen zwischen -20 °C und Raumtemperatur, vorzugsweise bei 0 °C, durchgeführt werden.

Die zu den Aldehyden der Formel VIII korrespondierenden primären Alkohole sind bekannt oder können durch an sich bekannte Verfahren aus bekannten Vorläuferverbindungen hergestellt werden. Beispielsweise können die primären Alkohole durch an sich bekannte Reduktionsverfahren, beispielsweise durch Reduktion mit komplexen Alkalimetallhydriden wie Lithiumaluminiumhydrid, aus den entsprechenden freien Aminocarbonsäure-Vorläuferverbindungen hergestellt werden. Vorzugsweise sind Aminocarbonsäuren geeignet, welche bereits in isomerenreiner, beispielsweise enantiomerenreiner Form vorliegen, wie die an sich bekannten natürlich vorkommenden 20 proteinogenen α-Aminosäuren. Ebenso können kommerziell erhältliche, beispielsweise von der Firma ChiroTech, Cambridge, (Katalog "The ChiroChem™ Collection, Series 1, FMOC unnatural amino acids for medicinal and combinatorial chemists", SCRIP Nr. 2311/20.02.1998, Seite 15) erhältliche nicht-natürliche isomerenreine α-Aminosäuren verwendet werden. Zur Herstellung von Verbindungen der Formel I, worin n = 1 ist, kann zweckmäßigerweise von an sich, beispielsweise aus Nohira et al., Bulletin of the Chemical Society of Japan 43 (1970) 2230 ff., bekannten isomerenreinen β-Aminosäuren ausgegangen werden. Weiterhin können für die Erfindung geeignete isomerenreine β-Aminosäuren auch aus isomerenreinen α-Aminosäuren durch Homologisierung, beispielsweise durch Homologisierung nach Arndt-Eistert gemäß den Methoden von D. Seebach et al., Helvetica Chimica Acta (= HCA) 79 (1996) 913-941; 2043 ff. und Synlett (1997) 437 ff., hergestellt werden. α-chirale β-Aminosäuren, worin R⁵ eine andere Bedeutung als Wasserstoff besitzt, können auf an sich bekannte Weise, beispielsweise durch asymmetrische Alkylierung von chiralen Oxazolidinonen mit Chlormethylamiden nach der Methode von D. Seebach et al., Synlett (1997) 437 ff., oder auch nach anderen, an sich bekannten Methoden erhalten werden.

Die gewünschten Schutzgruppen R¹³ können nach an sich bekannten Methoden in Verbindungen der Formel VIII oder deren vorstehend genannten Vorläuferverbindungen eingeführt werden.

Im Verfahrensschritt a) entstehen durch die Reaktion zwischen einem chiralen Aminoaldehyd der Formel VIII und dem aus einem 2-Alkenylsulfoximid der Formel II durch Deprotonierung und Transmetallierung entstandenen chiralen Zwischenprodukt in den Vinylsulfoximiden der Formel IX zwei neue stereogene Kohlenstoffatome. Diese neuen stereogenen Kohlenstoffatome sind die Atome C-3 und C-4 in Verbindungen der Formel IX. Die Substituenten R⁴ an C-4 und OM²(OR¹²)₃ an C-3 nehmen bei der Bildung der Vinylsulfoximide der Formel IX nach dem erfindungsgemäßen Verfahren in der Regel mit hoher Selektivität von mindestens 95 % eine "anti"-Orientierung zueinander ein. Die Absolutkonfigurationen an den neu entstehenden Chiralitätszentren C-3 und C-4 werden hierbei während der Reaktion jeweils durch die Absolutkonfiguration am Schwefelatom in Verbindungen der Formel II im Sinne einer regio- und diastereokontrollierten Reaktion gesteuert. Sofern das Schwefelatom in Verbindungen der Formel II R-Konfiguration besitzt, wird die prochirale Carbonylgruppe in den Aldehyden der Formel VIII von der Si-Seite angegriffen. Sofern dagegen das Schwefelatom in Verbindungen der Formel II S-Konfiguration besitzt, wird die prochirale Carbonylgruppe in den Aldehyden der Formel VIII von der Re-Seite angegriffen. Durch die auf diese Weise festgelegte absolute Konfiguration der Verbindungen der Formel IX ist auch die Stereochemie der Verbindungen der Formeln Ia, Ib und Ic an den entsprechenden Chiralitätszentren als eine "cis"-Orientierung festgelegt. Die Absolutkonfiguration am chiralen Kohlenstoffatom eines Aminoaldehyds der Formel VIII hat kaum Einfluß auf die Stereochemie an den Kohlenstoffatomen C-3 und C-4 der Verbindungen der Formel IX.

Die Behandlung von Verbindungen der Formel IX mit einem zur Abspaltung der Schutzgruppe R¹³ geeigneten Reagenz im Verfahrensschritt b) um Verbindungen der Formel Xa zu erhalten, kann direkt im Anschluß an Verfahrensschritt a) in situ auf an sich bekannte Weise erfolgen, ohne daß eine Isolierung der Verbindungen der Formel IX notwendig ist. Die Reaktion kann demgemäß in vorstehend angegebenen Lösungsmitteln und bei vorstehend angegebenen Temperaturen zwischen -100 °C und -50 °C, vorzugsweise bei -78 °C, durchgeführt werden. Basenlabile Schutzgruppen können beispielsweise mit an sich bekannten, in dem Reaktionsgemisch löslichen nicht-nucleophilen oder schwach nucleophilen organischen Basen abgespalten werden. Sofern die FMOC-Gruppe als Aminoschutzgruppe R¹³ verwendet wird, ist Piperidin als Base zu deren Abspaltung bevorzugt. Üblicherweise wird die Base in einer überstöchiometrischen Menge eingesetzt, beispielsweise im Molverhältnis von etwa 5:1 bis etwa 15:1, vorzugsweise von etwa 10:1, bezogen auf die eingesetzte Menge an aus Verbindungen der Formel II entstandenen Verbindungen der Formel IX. Nach erfolgter Zugabe der Base kann zuerst auf 0 °C, später auf Raumtemperatur, aufgetaut werden und das Reaktionsgemisch kann in üblicher Weise aufgearbeitet werden, wobei gegebenenfalls entstandene Nebenprodukte auf an sich bekannte Weise, beispielsweise durch Kristallisieren und/oder Chromatographie abgetrennt werden können.

Durch die Abspaltung der Aminoschutzgruppe R¹³ aus Verbindungen der Formel IX, vorzugsweise durch deren baseninduzierte Abspaltung, wird eine Ringschlußreaktion zu Verbindungen der Formel Xa eingeleitet. Insbesondere für Verbindungen der Formel IX, worin R⁴ nicht für Wasserstoff steht, verläuft die Cyclisierungsreaktion in der Weise, daß der Sulfonimidoylrest in 5-Position der entstehenden Verbindung der Formel Xa vorzugsweise die "trans"-Stellung zur Hydroxylgruppe in 3-Position des entstehenden Ringgerüstes einnimmt.

In entstandenen Azacyclen, welche ein sekundäres Ring-Stickstoffatom enthalten, kann anschließend dieses Stickstoffatom auf an sich bekannte Weise mit einer Verbindung, welche eine zur Reaktion mit einem sekundären Amin geeignete Gruppe enthält, weiter umgesetzt werden. Beispielsweise kann eine Umsetzung des Stickstoffatoms mit an sich bekannten Carbonsäuren zur Ausbildung von Peptidbindungen erfolgen. Ebenso kann das vorgenannte Stickstoffatom auch auf an sich bekannte Weise, beispielsweise durch Umsetzung mit einem Alkylhalogenid wie einem Phenylniederalkylhalogenid, beispielsweise Benzylchlorid, alkyliert werden. Nach diesen vorstehend beschriebenen Methoden oder auf andere an sich bekannte Weise, kann das Stickstoffatom auch mit einer üblichen Aminoschutzgruppe, vorzugsweise einer basenstabilen Schutzgruppe, blokkiert werden. Insbesondere ist es vorteilhaft, das Ringstickstoffatom in Verbindungen der Formel Xa mit einer basenstabilen Schutzgruppe zu blockieren, wenn Verbindungen der Formel Ib hergestellt werden sollen. Als basenstabile Schutzgruppe eignen sich vorzugsweise ein Carbamat bildende Schutzgruppen, insbesondere die tert-Butyloxycarbonyl-Schutzgruppe (= BOC).

Aus Verbindungen der Formel Xa können allfällige Schutzgruppen gewünschtenfalls auch auf an sich bekannte Weise, gegebenenfalls selektiv, wieder abgespalten werden. So kann es insbesondere vorteilhaft sein, aus Verbindungen der Formel Xa eine gegebenenfalls nach dem Verfahrensschritt b) noch vorhandene Silylschutzgruppe R¹¹ vor der Umsetzung mit einem zur reduktiven Spaltung der Sulfonimidoyl-Alkyl-Bindung geeigneten Reagenz im Verfahrensschritt ca) auf an sich bekannte Weise abzuspalten, sofern diese Abspaltung der Silylschutzgruppe im Verfahrensschritt b) nicht spontan geschehen ist. Als Beispiel für eine Silylschutzgruppe, welche im Verfahrensschritt b) üblicherweise spontan abgespalten wird, ohne daß es einer zusätzlichen Behandlung bedarf, sei Trimethylsilyl (= TMS) genannt.

Verbindungen der Formel Xa oder aus Verbindungen der Formel Xa durch Abspaltung von Schutzgruppen erhältliche Verbindungen sind neue Verbindungen mit nützlichen Eigenschaften und können beispielsweise als Zwischenprodukte zur Herstellung von Verbindungen der Formel I dienen. Das (2S,3R,4R,5R,S_{S})-2-Benzyl-3-hydroxy-5-{N-[(S)-1-hydroxy-3-methylbut-2-yl]-4-methylphenylsulfonimidoylmethyl}-4-methyl-1-(4-methylphenylsulfonyl)pyrrolidin ist bereits bekannt aus der Veröffentlichung im Internet unter der Adresse "www.iucr.ac.uk" von M. Bolte, Acta Crystallographica Section C, electronically published paper QA0019 [=(IUCr) Acta C Paper QA0019]. In der angegebenen Veröffentlichung ist jedoch kein Verfahren zur Herstellung dieser Verbindung angegeben.

Die reduktive Spaltung der Sulfonimidoyl-Alkyl-Bindung in einer erhaltenen Verbindung der Formel Xa oder in einer aus einer Verbindung der Formel Xa durch die vorstehend beschriebenen Umsetzungen am Ring-Stickstoffatom erhaltenen Verbindung im Verfahrensschritt ca) zur Herstellung von Verbindungen der Formel Ib, kann in einem vorstehend für die Umsetzung von Verbindungen der Formel II mit Verbindungen der Formel VII angegebenen polaren oder schwach polaren Lösungsmittel oder in Gemischen dieser Lösungsmittel durchgeführt werden. Vorzugsweise kann THF verwendet werden. Die Reaktion kann bei Temperaturen zwischen -20 °C und Raumtemperatur, vorzugsweise bei 0 °C ausgeführt werden. Als Reagenzien zur Spaltung der Sulfonimidoyl-Alkyl-Bindung eignen sich beispielsweise Reduktionsmittel wie Raney-Nickel, Lithiumnaphthalenid oder Samarium-(II)-iodid. Vorzugsweise kann Samarium-(II)-iodid eingesetzt werden.

Sofern die Desulfurierung mit Samarium-(II)-iodid durchgeführt wird, kann dieses auf an sich bekannte Weise in situ aus Samarium und Diiodmethan erzeugt werden. Üblicherweise wird hierbei das Samarium-(II)-iodid in einer überstöchiometrischen Menge, beispielsweise in einem Molverhältnis von etwa 3:1 bis etwa 7:1, bezogen auf die eingesetzte Verbindung der Formel Xa, verwendet. Zur Durchführung der Reaktion gibt man dem Reaktionsgemisch aus Verbindung der Formel Xa und Samariumdiiodid eine Protonenquelle, wie eine in dem verwendeten Lösungsmittel lösliche protische Verbindung, in einer geeigneten Menge zu. Als Protonenquelle kann beispielsweise ein niederer Alkohol wie Methanol verwendet werden. Vorzugsweise wird wasserfreies Methanol verwendet. Eine geeignete Menge der Protonenquelle kann beispielsweise zwischen 2 und 5 Äquivalente, bezogen auf ein Äquivalent der in einer Verbindung der Formel Xa enthaltenen Menge an Schwefel, betragen. Besonders vorteilhaft können hierbei Verbindungen der Formel Xa eingesetzt werden, worin ein sekundäres Ringstickstoffatom durch eine Carbamat-Schutzgruppe, vorzugsweise die BOC-Schutzgruppe, blockiert ist.

Die Spaltung der Sulfonimidoyl-Alkyl-Bindung unter den Bedingungen einer baseninduzierten reduktiven Eliminierung in einer erhaltenen Verbindung der Formel Xa, worin R¹⁰¹ nicht Wasserstoff bedeutet, oder in einer aus einer Verbindung der Formel Xa durch die vorstehend beschriebenen Umsetzungen am Ring-Stickstoffatom erhaltenen Verbindung im Verfahrensschritt ca) zur Herstellung von Verbindungen der Formel Ic, kann in einem vorstehend für die Umsetzung von Verbindungen der Formel II mit Verbindungen der Formel VII angegebenen polaren oder schwach polaren Lösungsmittel oder auch in einem teilhalogenierten Niederalkyl-Lösungsmittel wie Dichlormethan durchgeführt werden. Vorzugsweise kann Dichlormethan verwendet werden. Als Basen zur Spaltung der Sulfonimidoyl-Alkyl-Bindung durch β-Eliminierung eignen sich nicht-nucleophile organische Basen wie bicyclische Amidine, beispielsweise 1,5-Diazabicyclo[4.3.0]-5-nonen (= DBN) oder 1,8-Diazabicyclo[5.4.0]-7-undecen (= DBU). Vorzugsweise kann DBU verwendet werden. Zweckmäßigerweise wird die Reaktion so ausgeführt, daß die Sulfonimidoyl-Gruppe einer oben angegebenen Verbindung der Formel Xa auf an sich bekannte Weise elektrophil aktiviert wird. Hierzu kann die Verbindung der Formel Xa bei Temperaturen zwischen -25 °C und -15 °C mit einer zur Bildung einer guten Abgangsgruppe aus der Sulfonylgruppe geeigneten Verbindung oder mit einem Niederalkyloxonium-Tetrafluoroborat wie dem als "Meerwein-Salz" bekannten Trimethyloxonium-tetrafluoroborat, umgesetzt werden. Reagenzien, welche durch Angriff an der Sulfonylgruppe eine gute Abgangsgruppe bilden können, sind beispielsweise Ester oder Halogenide von Sulfonsäuren wie Methansulfonsäurechlorid, Trifluormethansulfonsäurechlorid, Trifluormethansulfonsäure-Methylester (= Methyl-Triflat) oder Trifluormethansulfonsäure-Trimethylsilylester (= TMS-Triflat). Vorzugsweise kann Methyl-Triflat eingesetzt werden. Üblicherweise läßt man das entstandene Reaktionsgemisch nach erfolgter Umsetzung auf Raumtemperatur auftauen und gibt anschließend die vorgenannte Base zu.

In den erhaltenen Verbindungen der Formel Ia ist die im Verfahrensschritt b) durch Ringschluß zu Verbindungen der Formel Xa entstandene relative Orientierung des Sulfonimidoyl-Substituenten in 5-Position und der Hydroxylgruppe in 3-Position als eine "trans"-Orientierung zueinander festgelegt. Verbindungen der Formel I, worin der Substituent YH in 3-Position Hydroxy oder Amino bedeuten kann und/oder worin die Substituenten YH in 3-Position und R¹-CHR²- in 5-Position auch in "cis"-Orientierung zueinander stehen können, können gewünschtenfalls aus Verbindungen der Formel Ia durch eine ein- oder mehrmals durchgeführte, unter Inversion verlaufende, nucleophile Substitutionsreaktion am Ringkohlenstoffatom in 3-Position erhalten werden. Derartige nucleophile Substitutionsreaktionen sind an sich bekannt und können beispielsweise unter den Bedingungen einer Mitsunobu-Reaktion durchgeführt werden (siehe z. B. Mitsunobu, Synthesis 1 (1981) 1 - 28).

Sofern beispielsweise Verbindungen der Formel I gewünscht sind, worin YH für Hydroxy steht und worin die Substituenten OH in 3-Position und R¹-CHR²- in 5-Position in "cis"-Orientierung zueinander stehen, kann zweckmäßig eine Mitsunobu-Reaktion in der Weise ausgeführt werden, daß man eine Lösung von einer Verbindung der Formel Ia, worin allenfalls weitere vorhandene Hydroxylgruppen durch Schutzgruppen blockiert sind, und von Triphenylphosphin, in einem unter den Reaktionsbedingungen inerten organischen Lösungsmittel wie einem cyclischen oder offenkettigen Niederalkylether, beispielsweise Diethylether oder THF, zu einer Vorlage einer Lösung von Diethylazodicarboxylat (= DEAD) und einer Säure, beispielsweise Phosphorsäure oder eine Carbonsäure wie Benzoesäure, hinzugegeben werden. Die Reaktion kann vorzugsweise bei Raumtemperatur ausgeführt werden. Der auf diese Weise erhaltene Ester einer gewünschten Verbindung der Formel I kann gewünschtenfalls anschließend noch auf an sich bekannte Weise gespalten werden, um die freie Hydroxylgruppe in der 3-Position zu erhalten.

Sofern beispielsweise Verbindungen der Formel I gewünscht sind, worin Y für NH steht und worin die Substituenten Amino in 3-Position und R¹-CHR²- in 5-Position in "cis"-Orientierung zueinander stehen, kann zweckmäßig eine Mitsunobu-Reaktion in der Weise ausgeführt werden, daß man eine Lösung von DEAD in einem vorstehend genannten inerten Lösungsmittel zu einer Vorlage einer Lösung von Triphenylphosphin, einer Verbindung der Formel Ia, worin allenfalls weitere vorhandene Hydroxylgruppen durch Schutzgruppen blockiert sind, und einem zur nucleophilen Substitution einer Hydroxylgruppe durch eine Aminogruppe in aliphatischen Resten geeigneten Reagenz wie Phthalimid, zugibt. Das entstandene Zwischenprodukt, beispielsweise ein N-substituiertes Phthalimid, kann dann in einem protischen Lösungsmittel wie einem niederen Alkanol, beispielsweise Ethanol, mit einem zur Freisetzung des entstandenen Amins der Formel I geeigneten Reagenz wie Hydrazin behandelt werden.

Sofern beispielsweise Verbindungen der Formel I gewünscht sind, worin Y für NH steht und worin die Substituenten YH in 3-Position und R¹-CHR²- in 5-Position in "trans"-Orientierung zueinander stehen, kann in einer vorstehend angegebenen Verbindung der Formel Ia zuerst eine oben beschriebene Inversion des Ringkohlenstoffatoms in 3-Position unter Erhalt des Hydroxy-Substituenten durchgeführt werden und an diesem Zwischenprodukt der Formel I kann dann noch eine oben beschrieben Substitution der Hydroxylgruppe durch eine Aminogruppe unter erneuter Inversion des Ringkohlenstoffatoms in 3-Position durchgeführt werden.

Die erhaltenen Verbindungen der Formel I können auf an sich bekannte Weise aus dem Reaktionsgemisch isoliert werden. Allfällige Schutzgruppen können gewünschtenfalls auf an sich bekannte Weise, gegebenenfalls selektiv, wieder abgespalten werden und die Gruppe YH kann gewünschtenfalls mit an sich bekannten Schutzgruppen blockiert werden. Die gegebenenfalls freigesetzte NH-Gruppe in 1-Position des cyclischen Grundgerüstes kann gewünschtenfalls mit vorstehend genannten, zur N-Alkylierung oder zur Amidbildung befähigten, Reagenzien umgesetzt oder mit einer Aminoschutzgruppe blockiert werden. Gewünschtenfalls können Verbindungen der Formel I, welche basische Aminogruppen enthalten, auf an sich bekannte Weise in Säureadditionssalze überführt werden. Als Säuren eignen sich hierfür beispielsweise Mineralsäuren wie Salzsäure oder Schwefelsäure, oder organische Säuren wie Sulfonsäuren, beispielsweise Methylsulfonsäure oder p-Toluolsulfonsäure, oder Carboxylsäuren wie Essigsäure, Trifluoressigsäure, Weinsäure oder Citronensäure.

Die Verbindungen der allgemeinen Formeln Ia, Ib und Ic sind neue Verbindungen und stellen wertvolle Ausgangsstoffe, beispielsweise zur Herstellung chiraler Katalysatoren für die asymmetrische Synthese, für die Herstellung biologisch aktiver Alkaloide oder Porphyrine sowie für die Herstellung pharmakologisch interessanter Verbindungen dar.

Die Ausgangsverbindungen der Formel II können auf an sich bekannte Weise hergestellt werden.

Beispielsweise können Verbindungen der allgemeinen Formel IIa 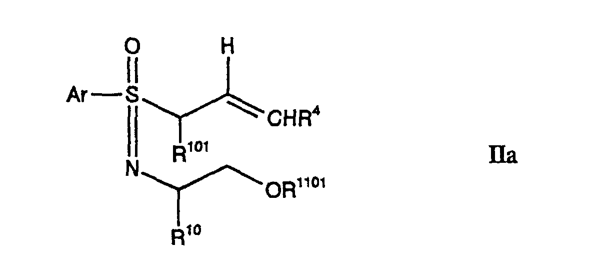 worin R¹⁰¹, R⁴, R¹⁰, R¹¹⁰¹ und Ar obige Bedeutungen besitzen, hergestellt werden, indem man ein Stereoisomer einer Verbindung der allgemeinen Formel III, 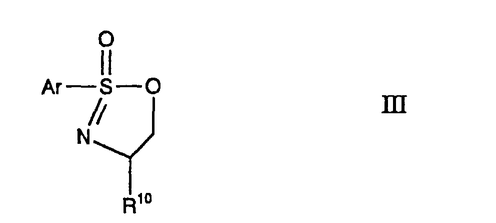 worin Ar und R¹⁰ obige Bedeutungen besitzen, mit einer Verbindung der allgemeinen Formel IV, 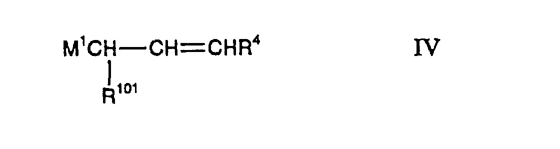 worin R¹⁰¹ und R⁴ obige Bedeutungen besitzen und M¹ für eine einwertige, ein Alkalimetall oder ein Erdalkalimetall und ein Halogenatom enthaltende Gruppe steht, umsetzt und eine bei dieser Umsetzung allenfalls freiwerdende Hydroxylgruppe mit einer Silyl-Schutzgruppe R¹¹⁰¹ blockiert.

Die Umsetzung eines Stereoisomers von cyclischen Sulfonimidaten der Formel III mit einem metallierten Alken der Formel IV zu einem isomerenreinen 2-Alkenylsulfoximid der Formel II kann in einem vorstehend für die Umsetzung von Verbindungen der Formel II mit Verbindungen der Formel VII angegebenen polaren oder schwach polaren aprotischen Lösungsmittel durchgeführt werden. Vorzugsweise kann THF verwendet werden. Die Reaktion kann ausgeführt werden, indem man die Reaktanden bei einer Temperatur von -100 °C bis -50 °C, vorzugsweise bei - 78 °C, in einem vorstehend angegebenen Lösungsmittel vermischt und die entstandene Reaktionsmischung kurze Zeit, z. B. 2 bis 10 Minuten, bei der angegebenen Temperatur reagieren läßt und anschließend auf eine höhere Temperatur unterhalb der Raumtemperatur, beispielsweise auf -20 °C bis 0 °C, erwärmen läßt. Nötigenfalls kann zur Vervollständigung der Reaktion noch einige Zeit bei -20 °C bis 0 °C weitergerührt werden. Vorteilhaft ist es, die Verbindung der Formel IV in überstöchiometrischen Mengen einzusetzen. Beispielsweise können 1,5 bis 2,5 Mol einer Verbindung der Formel IV mit einem Mol einer Verbindung der Formel III umgesetzt werden.

In den cyclischen Sulfonimidaten der Formel III kann Ar vorzugsweise für 4-Methylphenyl (= p-Tolyl) stehen. R¹⁰ kann insbesondere Methyl, Isopropyl, Isobutyl oder Phenyl bedeuten und steht vorzugsweise für Isopropyl.

Um eine gewünschte stereochemisch kontrollierte Herstellung der Verbindungen der Formel I zu erzielen, sollten die Sulfonimidate der Formel III in isomerenreiner Form verwendet werden. Als isomerenrein soll im Rahmen der vorliegenden Erfindung grundsätzlich ein Isomerenüberschuß (= Enantiomerenüberschuß, ee oder Diastereoisomerenüberschuß, de) eines reinen Isomers von mindestens 95 % verstanden werden. In den im Rahmen der vorliegenden Erfindung angegebenen Formeln bezeichnet das "*"-(Sternchen) Zeichen jeweils ein Chiralitätszentrum, welches üblicherweise isomerenrein entsteht oder aus üblicherweise isomerenrein eingesetzten Edukten stammt. Sofern nicht-isomerenreine, beispielsweise racemische, Ausgangsverbindungen zur Herstellung von Verbindungen der Formel I verwendet werden, können nach dem erfindungsgemäßen Herstellungsverfahren natürlich auch Isomerengemische von Verbindungen der Formel I erhalten werden. Sofern Sulfonimidate der Formel III eingesetzt werden, worin das chirale Schwefelatom und das den Substituenten R¹⁰ tragende chirale Kohlenstoffatom unterschiedliche Absolutkonfigurationen aufweisen (d. h. wenn z. B. das Schwefelatom R-Konfiguration besitzt und das den Substituenten R¹⁰ tragende Kohlenstoffatom S-Konfiguration aufweist), werden besonders gute Ergebnisse bezüglich der stereochemischen Reinheit der Produkte der Formel I erzielt. Besonders bevorzugt können als Verbindungen der Formel III das (R_{S})-4(R)-Isopropyl-2-p-tolyl-4,5-dihydro-[1,2λ⁶, 3]oxathiazol-2-oxid und das (S_{S})-(4R)-Isopropyl-2-p-tolyl-4,5-dihydro-[1,2λ⁶,3]-oxathiazol-2-oxid, (Rₛ)-4(R)-Isopropyl-2-p-toluoyl-4,5-dihydro[1,2λ⁶,3]oxathiazol-2-oxid und (S_{S})-4(R)-Isopropyl-2-p-toluoyl-4,5-dihydro[1,2λ⁶,3]-oxathiazol-2-oxid verwendet werden. Die Ausdrücke R_{S} und S_{S} bezeichnen jeweils die Absolutkonfiguration am chiralen Schwefelatom. Sulfonimidate der Formel III sind beispielsweise bekannt aus Reggelin et al., Tetrahedron Letters (= TL) 33 (1992) 6959-6962 oder aus Reggelin et al., TL 36 (1995) 5885-5886 und können nach den dort jeweils angegebenen oder dazu analogen Verfahren isomerenrein hergestellt werden.

In den metallierten Verbindungen der Formel IV kann die einwertige Gruppe M¹ ein Alkalimetall, vorzugsweise Lithium, oder eine ein Erdalkalimetall und zusätzlich ein Halogenatom enthaltende Gruppe bedeuten. Als Erdalkalimetall ist Magnesium bevorzugt. Als Halogen kann Chlor, Brom oder Iod eingesetzt werden. Insbesondere können als metallierte Verbindungen der Formel IV an sich bekannte lithiierte Alkenylverbindungen oder an sich bekannte magnesiumorganische Alkenylverbindungen wie Alkenyl-Grignard-Reagenzien verwendet werden.

Üblicherweise wird eine bei der Umsetzung von Verbindungen der Formel III mit Verbindungen der Formel IV zu Verbindungen der Formel IIa freiwerdende Hydroxylgruppe mit einer geeigneten Silyl-Schutzgruppe R¹¹⁰¹ blockiert, um unerwünschte Folgereaktionen zu verhindern. Als Silyl-Schutzgruppe R¹¹⁰¹ in Verbindungen der Formel IIa kann vorzugsweise Trimethylsilyl (= TMS) verwendet werden.

Verbindungen der allgemeinen Formel IIb, 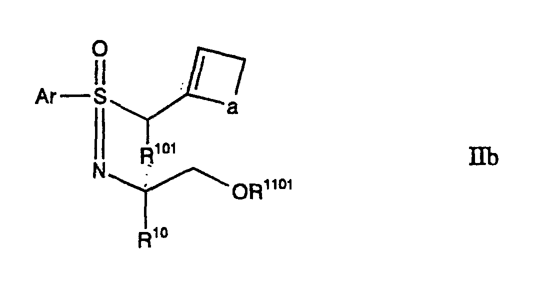 worin R¹⁰¹, R¹⁰, R¹¹⁰¹ und Ar obige Bedeutungen besitzen und a Methylen bedeutet oder eine C₂-C₅-Alkylenkette bedeutet, welche durch gegebenenfalls ein- oder zweifach durch niederes Alkyl substituiertes C₁-C₂-Alkylen überbrückt sein kann, können beispielsweise hergestellt werden, indem man ein Stereoisomer einer Verbindung der allgemeinen Formel V, 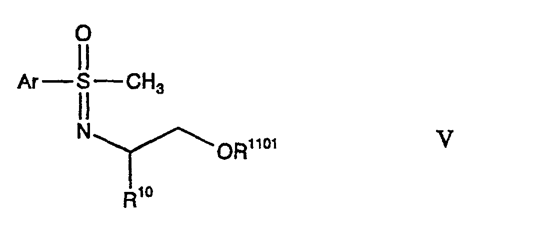 worin R¹⁰, R¹¹⁰¹ und Ar obige Bedeutungen besitzen, mit einer zu deren Deprotonierung geeigneten Base deprotoniert, die deprotonierte Verbindung der Formel V mit einer Verbindung der allgemeinen Formel VI, 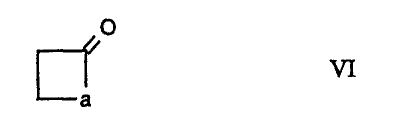 worin a obige Bedeutung besitzt, umsetzt, und das erhaltene Zwischenprodukt nacheinander mit einem Reagenz, welches die Abspaltung des aus der Carbonylgruppe der Verbindung der Formel VI stammenden Sauerstoffatoms ermöglicht und mit einer vorstehend angegebenen, zur Deprotonierung einer Verbindung der Formel V geeigneten Base behandelt.

Die Reaktionsfolge zur Herstellung von Cycloalkenylmethyl-Sulfoximid-Verbindungen der Formel IIb durch Umsetzung von Verbindungen der Formel V mit Verbindungen der Formel VI kann zweckmäßig als Eintopf-Reaktionssequenz durchgeführt werden. Die Umsetzung eines Stereoisomers eines Methylsulfoximids der Formel V mit einer zu dessen Deprotonierung geeigneten Base sowie die nachfolgenden Reaktionsschritte: Umsetzung der deprotonierten Verbindung der Formel V mit einer Verbindung der Formel VI, Behandlung des erhaltenen Zwischenproduktes mit einem Reagenz, welches die Abspaltung des aus der Carbonylgruppe der Verbindung der Formel VI stammenden Sauerstoffatoms ermöglicht und erneute Behandlung mit einer vorstehend angegebenen Base, sind an sich bekannt und können nach einem in Reggelin et al., JACS 118 (1996) 4765-4777, angegebenen oder hierzu analogen Verfahren durchgeführt werden. Die Gruppe Ar sowie der Substituent R¹⁰ in Verbindungen der Formel V können die vorstehend für Verbindungen der Formel III angegebenen bevorzugten Bedeutungen besitzen. Als Silyl-Schutzgruppe R¹¹⁰¹ in Verbindungen der Formel V kann vorzugsweise tert.-Butyldimethylsilyl (= TBS) verwendet werden. Analog zu den vorstehend für Verbindungen der Formel III angegebenen bevorzugten stereochemischen Gegebenheiten können als Verbindungen der Formel V vorzugsweise das [S_{S},N(1S)]-N-[1-[[tert.-Butyldimethylsilyl)oxy]methyl]-2-methylpropyl]-Smethyl-S-(4-methylphenyl)sulfoximid und das [R_{S},N(1R)]-N-[1-[[tert.-Butyldimethylsilyl)oxy]methyl]-2-methylpropyl]-S-methyl-S-(4-methylphenyl)sulfoximid eingesetzt werden. Als Basen zur Deprotonierung von Verbindungen der Formel V eignen sich beispielsweise lithiierte Niederalkylverbindungen wie n-Butyllithium. Als Reagenzien, welche die Abspaltung von aus der Carbonylgruppe von Verbindungen der Formel VI stammenden Sauerstoffatomen ermöglichen, sind die vorstehend zur Bildung einer guten Abgangsgruppe durch Angriff am Sauerstoffatom der Sulfonylgruppe in Verbindungen der Formel Xa genannten Verbindungen geeignet. Bevorzugt kann TMS-Triflat eingesetzt werden.

Die alicyclischen Ketone der Formel VI sind bekannt. Beispielsweise können Cyclopentanon, Cyclohexanon oder Nopinon als Verbindungen der Formel VI eingesetzt werden. Sofern überbrückte cyclische Ketone als Verbindungen der Formel VI eingesetzt werden, ist es vorteilhaft, wenn die überbrückende Alkylenkette wenigstens an eines der beiden in α-Stellung zur Carbonylgruppe stehenden Kohlenstoffatome gebunden ist. Auf diese Weise werden die Reaktionsprodukte stets unter kontrollierter Regioselektivität gebildet.

Eine andere Möglichkeit, Verbindungen der Formel IIb zu erhalten, ist die Umsetzung einer Verbindung der allgemeinen Formel XII, 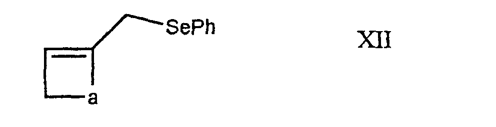 worin a und Ph obige Bedeutungen besitzen, jeweils mit einem zu deren lithiierender Deselenierung geeigneten Reagenz und die nachfolgende Umsetzung des jeweils entstandenen deselenierten lithiierten Zwischenproduktes mit einem Stereoisomer einer Verbindung der Formel III.

Die selenierten Verbindungen der Formel XII können auf an sich bekannte Weise aus den korrespondierenden Allylalkoholen durch Halogenierung und nachfolgende reduzierende Selenierung erhalten werden. Beispielsweise können die Verbindungen der Formeln XII nach dem von Reggelin et al. in JACS 118 (1996) 4765 - 4777 angegebenen oder hierzu analogen Verfahren erhalten werden. Als Beispiel für einen Allylalkohol, welcher sich zur Herstellung von selenierten Verbindungen der Formel XII eignet, sei Myrtenol genannt.

Die Herstellung von Verbindungen der Formel IIb durch Umsetzung von Verbindungen der Formel XII mit Verbindungen der Formel III kann auf an sich bekannte Weise durchgeführt werden, beispielsweise nach der in der Veröffentlichung von Reggelin et al., JACS 118 (1996) 4765-4777 angegebenen Methode zur Herstellung von Cycloalkenylsulfoximid-Verbindungen, auf die hiermit ausdrücklich Bezug genommen wird.

In einer Variante der Erfindung können Verbindungen der Formel II, worin R¹⁰¹ eine andere Bedeutung als Wasserstoff besitzt, hergestellt werden, indem man Verbindungen der Formel II, worin R¹⁰¹ für Wasserstoff steht, mit einer hierfür geeigneten Base einfach deprotoniert und anschließend durch Umsetzung mit einer Verbindung der allgemeinen Formel XI,

**R**^{**103**}**―Z XI**

worin R¹⁰³ die für R¹⁰¹ angegebene Bedeutung mit Ausnahme von Wasserstoff besitzt und Z für eine abspaltbare Fluchtgruppe steht, alkyliert. Als Basen für eine vorstehend angegebene Deprotonierung eignen sich beispielsweise lithiierte Niederalkylverbindungen wie n-Butyllithium. Als abspaltbare Fluchtgruppe Z in Verbindungen der Formel XI kann beispielsweise Halogen, vorzugsweise Brom oder Chlor, eingesetzt werden. Die Reaktion kann unter für diesen Reaktionstyp üblichen Reaktionsbedingungen durchgeführt werden.

Die nachfolgenden Beispiele sollen die Erfindung näher erläutern, ohne ihren Umfang zu beschränken.

Die Numerierung der Ringatome in den Beispielsverbindungen, insbesondere der chiralen Kohlenstoffatome, bezieht sich auf die in der allgemeinen Formel I angegebene Numerierung der Ringatome.

### Beispiel 1

### (+)-(2S,3S,4S,5S)-2-Tsobutyl-3-hydroxy-4,5-dimethyl-N-tert.-butoxycarbonyl-pyrrolidin

A) 6,0 g FMOC-Amino-geschützes S-2-Amino-4-methylpentanol (erhalten durch Lithiumaluminiumhydrid-Reduktion von Leucin) wurden unter Stickstoffatmosphäre und Wasserausschluß in 100 ml Dichlormethan suspendiert und auf 0 °C gekühlt. Zu dieser Vorlage gab man in einer Portion 10,0 g 1,1,1-Triacetoxy-1,1-dihydro-1,2-benziodoxol-3(1H)-on (= Periodinan) als Feststoff hinzu und rührte die entstandene Reaktionsmischung zwei Stunden lang bei Raumtemperatur. Anschließend wurde die Reaktionsmischung auf eine mit 100 ml Ether überschichtete Lösung aus 130 ml einer 10%igen wäßrigen Natriumthiosulfatlösung und 360 ml einer gesättigten wäßrigen Natriumhydrogencarbonatlösung gegossen. Man extrahierte die wäßrige Phase einmal mit 100 ml Ether, wusch die vereinigten organischen Phasen mit einer gesättigten wäßrigen Kochsalzlösung und trocknete über Natriumsulfat. Das Lösungsmittel wurde unter vermindertem Druck eingedampft und der auf diese Weise erhaltene rohe FMOC-geschützte S-2-Amino-4-methylvaleraldehyd wurde ohne weitere Reinigung für die folgende Reaktion eingesetzt.
   Zur Bestimmung der optischen Reinheit wurde ein Teil des erhaltenen Aldehyds durch Kristallisation aus Ether/-Hexan isoliert. Der Enantiomerenüberschuß wurde durch NMR-Spektroskopie unter Zugabe des chiralen Shift-Reagenzes Tris-[3-(heptafluoropropyl-hydroxymethylen)-dcamphorato]-praseodym (III) [= Pr(hfc)₃] bestimmt. Durch Integration der basisliniengetrennten Signale der Aldehydprotonen konnte der Enantiomerenüberschuß (ee) zu 95 % ermittelt werden.
B) 1,82 g Magnesiumspäne wurden mit etwa 10 ml Diethylether überschichtet und durch Zugabe von 500 mg frisch destilliertem Crotylbromid aktiviert. Zu dieser Vorlage tropfte man eine Lösung von 10,0 g Crotylbromid (= cis/trans-1-Brom-2-Buten) in 100 ml Diethylether bei 0 °C unter Argonschutz und Feuchtigkeitsausschluß langsam zu. Das entstandene Gemisch wurde nach erfolgter Zugabe noch 30 Minuten lang zum Sieden erhitzt. Die entstandene etherische Lösung von Crotylmagnesiumbromid wurde von nicht umgesetztem Magnesium abgetrennt und ohne weitere Aufarbeitung in Lösung direkt weiter umgesetzt.
   Zur Gehaltsbestimmung der vorstehend hergestellten Grignard-Lösung wurde eine Lösung von 180 mg (-)-Menthol und einer Spatelspitze Phenanthrolin in 3,0 ml THF auf 0 °C abgekühlt. Durch Zugabe der Grignard-Lösung zu dieser Vorlage titrierte man bis zum Farbumschlag nach Rot und ermittelte durch Differenzwägung die benötigte Menge an Grignard-Lösung für die folgende Umsetzung. Aus dem Quotienten der eingewogenen Menthol-Menge in mmol und der Masse der zur Titration bis zum Farbumschlag benötigten Grignard-Lösung in g ergibt sich der Gehalt der Grignard-Lösung in mmol/g.
C) Zu einer auf -40 °C gekühlten Lösung von 2,3g (+)-(R_{S})-4(R)-Isopropyl-2-p-tolyl-4,5-dihydro[1,2λ⁶,3]oxathiazol-2-oxid in 40 ml THF wurden unter Argonschutz und Feuchtigkeitsausschluß 46 g der vorstehend erhaltenen Lösung von Crotylmagnesiumbromid, gelöst in 100 ml Diethylether, zugetropft. Nach vollendeter Zugabe wurde noch fünf Minuten lang bei der angegebenen Temperatur gerührt, bevor man die Reaktionsmischung auf 0 °C erwärmen ließ. Man rührte weitere 45 Minuten bei dieser Temperatur und gab dann 50 ml einer gesättigten wäßrigen Ammoniumchloridlösung hinzu. Die organische Phase wurde abgetrennt, die wäßrige Phase zweimal mit Ether extrahiert und die vereinigten organischen Phasen wurden über Natriumsulfat getrocknet. Anschließend wurde das Lösungsmittel bei vermindertem Druck eingedampft und der Rückstand über Kieselgel chromatographiert (Laufmittel: anfangs Essigsäureethylester/n-Hexan 1:3 v/v, dessen Zusammensetzung kontinuierlich bis auf 3:1 verändert wurde). Man erhielt 1,4 g (R_{S},1R)-N-[1-(Hydroxymethyl)-2-methylpropyl]-S-(2-butenyl)-p-toluolsulfoximid als farbloses Öl, IR (Film) = 3440, 1220, 1115 cm⁻¹, optischer Drehwert [α]_{D}²⁰ = + 3,3° (c = 0,5 in Dichlormethan).
D) Zu einer auf 0 °C gekühlten Lösung von 1,4 g des vorstehend erhaltenen Sulfoximids und 0,7 ml Ethyldimethylamin in 13 ml Dichlormethan wurden unter Argonschutz und Feuchtigkeitsausschluß 0,6 ml Chlortrimethylsilan zugetropft. Nach vollendeter Zugabe wurde noch 15 Minuten lang bei 0 °C weitergerührt. Anschließend ließ man auf Raumtemperatur auftauen und goß das Reaktionsgemisch nach vollständiger Umsetzung auf eine Mischung aus 25 ml Ether und 25 g Eis. Man extrahierte die wäßrige Phase 3 mal mit je 10 ml Ether, vereinigte die organische Phasen und trocknete sie über Magnesiumsulfat. Das Lösungsmittel wurde unter vermindertem Druck eingedampft und der verbleibende Rückstand wurde durch Chromatographie an Kieselgel (Laufmittel: Ether/n-Hexan 1:1 v/v) gereinigt. Man erhielt 1,75 g (+)-(R_{S},1R)-N-[1-(Trimethylsilyloxymethylpropyl)-2-methyl]-S-(2-butenyl)-p-toluolsulfoximid als farbloses Öl, IR (Film) = 1240, 1080, 840 cm⁻¹, optischer Drehwert [α]_{D}²⁰ = + 15,5° (c = 1,0 in Dichlormethan).
E) Eine Lösung von 1,47 g des vorstehend erhaltenen TMS-geschützten 2-Alkenylsulfoximids in 8 ml Toluol wurde auf -78 °C gekühlt und unter Argonschutz und Wasserausschluß mit 2,75 ml einer 1,6-molaren Lösung von n-Butyllithium in n-Hexan versetzt. Man ließ die Reaktionsmischung 15 Minuten lang bei der angegebenen Temperatur rühren und gab anschließend 4,8 ml einer 1-molaren Lösung von Chlortris(isopropoxy)titan in n-Hexan hinzu. Es wurde weitere 5 Minuten lang bei -78 °C gerührt, auf 0 °C aufgetaut und noch 30 Minuten lang bei 0 °C gerührt. Anschließend kühlte man das Reaktionsgemisch wieder auf -78 °C. Zu dieser Vorlage gab man eine Lösung von 2,8 g des vorstehend unter A) erhaltenen Aminoaldehyds in 8 ml THF zu. Man ließ 60 Minuten lang bei -78 °C weiterrühren, gab 4 ml Piperidin hinzu und ließ auf 0 °C erwärmen. Nach 10 Stunden wurde das Reaktionsgemisch auf 120 ml einer mit 12 ml Essigsäureethylester (= EE) überschichteten, intensiv gerührten gesättigten Ammoniumcarbonatlösung gegossen. Man ließ dieses Gemisch 30 Minuten lang rühren und trennte anschließend die Phasen. Die organische Phase wurde mit 40 ml einer gesättigten Ammoniumchloridlösung gewaschen und die vereinigten wäßrigen Phasen wurden dreimal mit EE extrahiert. Die vereinigten organischen Phasen wurden über Natriumsulfat getrocknet und das Lösungsmittel wurde unter vermindertem Druck eingedampft. Den verbleibenden Rückstand nahm man mit einer Suspension von 0,6 g Kaliumcarbonat in 10 ml Methanol auf und ließ 60 Minuten lang rühren. Dann wurde von nicht gelöstem Kaliumcarbonat abfiltriert und das Filtrat wurde auf 4 °C abgekühlt. Man filtrierte von ausgefallenem Feststoff ab, wusch mit wenig 4 °C kaltem Methanol nach und dampfte das Filtrat bei vermindertem Druck ein. Der erhaltene Rückstand wurde in 5 ml Toluol aufgenommen und über Kieselgel filtriert (Laufmittel: anfangs Ether/Hexan 1:3 v/v dann EE). Die polare, pyrrolidinhaltige Fraktion wurde eingeengt und in 4 ml Dioxan aufgenommen. Zu dieser Vorlage gab man 1,0 g Di-tert.-butyl-dicarbonat [= (BOC)₂O] und eine Lösung von 0,7 g Natriumhydrogencarbonat in 8 ml Wasser zu. Man ließ 10 Stunden lang rühren, dampfte das Lösungsmittel unter vermindertem Druck ein und verteilte den verbleibenden Rückstand zwischen 5 ml Wasser und 10 ml Ether. Die wäßrige Phase wurde dreimal mit Ether extrahiert und die vereinigten organischen Phasen wurden über Natriumsulfat getrocknet. Nach erneutem Eindampfen des Lösungsmittels unter vermindertem Druck wurde der erhaltene Rückstand durch Chromatographie an Kieselgel (Laufmittel: Ether/Hexan 3:1 v/v) gereinigt. Man erhielt 1,0 g (R_{S},1'R,2S,3S,4S,5R)-N'-[(1-Hydroxymethyl)-2-(methylpropyl)]-S-4-hydroxy-3-methyl-2-(4-methylphenylsulfonimidoylmethyl)-5-isobutyl-N-tert.-butoxycarbonyl-pyrrolidin als farblosen Schaum, optischer Drehwert [α]_{D}²⁰ = -4° (c = 0,1 in Dichlormethan), IR (Film) = 3419, 1674, 1256, 1097 cm⁻¹.
F) Zu einer auf 0 °C gekühlten Suspension von 1,67 g Samarium in 40 ml THF wurden tropfenweise insgesamt 2,4 g Diiodmethan zugegeben. Nach erfolgter Zugabe wurde 15 Minuten lang bei 0 °C gerührt, bevor die Reaktionsmischung auf Raumtemperatur aufgetaut wurde. Man ließ weitere 60 Minuten lang bei Raumtemperatur rühren und gab dann eine Lösung von 1,0 g der vorstehend erhaltenen 2-Sulfonimidoylmethyl-Verbindung in einer Mischung aus 1,2 ml Methanol und 2,5 ml THF zu. Das Reaktionsgemisch wurde 4 Stunden lang gerührt und anschließend mit 110 ml gesättigter wäßriger Ammoniumchloridlösung versetzt. Nach der ersten Phasentrennung versetzte man die wäßrige Phase tropfenweise solange mit 0,5 N wäßriger Salzsäurelösung, bis die Phase aufklarte. Die wäßrige Phase wurde dreimal mit Ether extrahiert. Die vereinigten organischen Phasen wurden über Natriumsulfat getrocknet und das Lösungsmittel wurde unter vermindertem Druck eingedampft. Chromatographie des verbleibenden Rückstandes an Kieselgel (Laufmittel: Ether/n-Hexan 3:1 v/v) lieferte 0,5 g der Titelverbindung als farblosen Feststoff, Fp. = 97 °C, optischer Drehwert [α]_{D}²⁰ = + 66° (c = 1,0 in Dichlormethan).

### Beispiel 2

### (+)-(2S,3S,4S,5R)-3-Hydroxy-5-methyl-2-phenyl-(1-aza-N-tert.-butoxycarbonyl)-bicyclo[3.3.0]octan

A) Zu einer auf -78 °C gekühlten Lösung von 3,98 g (+)-R_{S}-4*R*-Isopropyl-2-*p*-tolyl-4,5-dihydro[1,2λ⁶,3]oxathiazol-2-oxid in 40 ml THF wurden unter Argonschutz und Feuchtigkeitsausschluß 16,6 ml einer 1,6 molaren Lösung von Methyllithium in Hexan zugetropft. Nach vollendeter Zugabe wurde noch fünf Minuten lang bei der angegebenen Temperatur gerührt, bevor man die Reaktionsmischung auf 0 °C erwärmen ließ. Man rührte weitere 45 Minuten bei dieser Temperatur und gab dann 160 ml Ammoniumchlorid hinzu. Nach dem Abtrennen der organischen Phase extrahierte man die wäßrige Phase noch zweimal mit 20 ml Ether und trocknete die vereinigten organischen Phasen über Natriumsulfat. Das Lösungsmittel wurde anschließend unter vermindertem Druck eingedampft. Der verbleibende Rückstand wurde bei Raumtemperatur in 80 ml Dichlormethan gelöst und hierzu wurden 3,8 g tert.-Butyldimethylsilylchlorid, 0,6 g *N,N*-Dimethylaminopyridin und 2,4 g Ethyldimethylamin zugegeben und es wurde 18 Stunden lang gerührt. Dann goß man die Mischung auf 40 ml Eiswasser, trennte die organische Phase ab und extrahierte die wäßrige Phase dreimal mit je 20 ml Dichlormethan. Nach dem Trocknen der vereinigten organischen Phasen über Natriumsulfat wurde das Lösungsmittel unter vermindertem Druck eingedampft. Reinigung des Rückstandes über Kieselgel (Laufmittel: Ether/n-Hexan = 1:1 v/v) lieferte 6,0 g (-)-R_{S}-N(1*R*)-*N-*[1-((tert.-Butyldimethylsilyl)oxy)-methyl-2-methylpropyl]-*S*-methyl-*S*-(4-methylphenyl)sulfoximid als farbloses Öl, optischer Drehwert [α]_{D}²⁰ = - 43,2° (c = 0,8 in Dichlormethan); IR (Film) = 1230, 1130 cm⁻¹.
B) Zu einer auf -78 °C gekühlten Lösung von 6,5 g des vorstehend erhaltenen Methylsulfoximids in 45 ml Toluol wurden unter Argonschutz und Feuchtigkeitsausschluß 12,45 ml einer 1,6-molaren Lösung von n-Buthyllithium in n-Hexan zugetropft. Man rührte 15 Minuten bei der angegebenen Temperatur und tropfte anschließend unverdünnt 2,2 g Cyclopentanon hinzu. Nach 10 Minuten ließ man die Reaktionsmischung auf Raumtemperatur erwärmen. Es wurden weitere 30 Minuten bei dieser Temperatur gerührt, bevor man den Ansatz auf -78 °C kühlte und 9,2 g Trimethylsilyltrifluormethylsulfonat hinzutropfte. Nach fünf Minuten wurde auf Raumtemperatur erwärmt und weitere drei Stunden gerührt. Nachdem erneut auf -78 °C gekühlt worden war, wurden 24,9 ml einer 1,6-molaren Lösung von n-Buthyllithium in n-Hexan zugetropft. Nach drei Minuten Rühren bei der angegebenen Temperatur ließ man auf Raumtemperatur auftauen und rührte noch 18 Stunden lang. Man goß die Reaktionsmischung auf 160 ml einer gesättigten wäßrigen Ammoniumchloridlösung, extrahierte zweimal mit Essigester und trocknete die vereinigten organischen Phasen über Natriumsulfat. Das Lösungsmittel wurde unter vermindertem Druck eingedampft und der verbleibende Rückstand wurde über Kieselgel (Laufmittel: Ether/n-Hexan 1:6 v/v) gereinigt. Man erhielt 5,5 g (-)-*R*_{*S*}*-N*(1*R*)-*N*-[1-((tert.-Butyldimethylsilyl)oxy)methyl-2-methylpropyl]-*S*-cyclopent-1-en-1-ylmethyl)-*S*-(4-methylphenyl)sulfoximid als farbloses Öl, optischer Drehwert [α]_{D}²⁰ = -2,5° (c = 1,6 in Dichlormethan), IR (Film) = 1240, 1120 cm⁻¹.
C) Auf die in vorstehend unter 1E) beschriebene Weise wurde eine Lösung von 2,95 g des vorstehend erhaltenen Cyclopentenylsulfoximids in 21 ml Toluol mit 4,8 ml einer 1,6-molaren Lösung von n-Buthyllithium in n-Hexan, 8,3 ml einer 1-molaren Lösung von Chlortris(isopropoxy)titan in n-Hexan, einer Lösung von 5,0 g FMOC-geschütztem S-α-Aminophenylethanal in 40 ml THF und 7 ml Piperidin umgesetzt. Chromatographie an Kieselgel (Laufmittel: Ether/n-Hexan = 1:3 v/v) lieferte 3,9 g (2S,3S,4S, 5R)-*R*_{*S*}-*N*(1*R*)-*N*-[1-((tert.-Butyldimethylsilyl)oxy)methyl-2-methylpropyl]-3-hydroxy-2-phenyl-5-(4-methylphenylsulfonimidoylmethyl-2-azabicyclo-[3.3.0]octan. Optischer Drehwert [α]_{D}²⁰ = +2,8°, (c = 0,6 in Dichlormethan); IR (Film) = 3443, 1251, 1103, 835 cm⁻¹.
D) Zu einer Lösung von 3,9 g des vorstehend erhaltenen Bicyclus in 20 ml Dichlormethan und 40 ml Wasser wurden 0,45 g Natriumhydrogencarbonat und 3,0 g Di-tert.-butyldicarbonat zugegeben und 12 Stunden gerührt. Nach Eindampfen des Lösungsmittels unter vermindertem Druck wurde der erhaltene Rückstand zwischen 5 ml Wasser und 10 ml Ether verteilt. Die organische Phase wurde abgetrennt und die wäßrige Phase zweimal mit Ether extrahiert. Trocknung der vereinigten organischen Phasen über Natriumsulfat, Eindampfen des Lösungsmittels unter vermindertem Druck und Chromatographie des verbleibenden Rückstandes an Kieselgel (Laufmittel: Ether/n-Hexan = 1:1 v/v) lieferte 4,39 g (-)-(2S,3S,4S,5S)-(-*R*_{*S*}-*N*(1*R*)-*N*-[1-((tert.butyldimethylsilyl)oxy)methyl-2-methylpropyl]-3-hydroxy-2-phenyl-5-(4-methylphenylsulfonimidoylmethyl-2-aza-(N-tert.-butoxycarbonyl)-bicyclo[3.3.0]octan, optischer Drehwert [α]_{D}²⁰ = -6,2° (c = 0,9 in Dichlormethan); IR (Film) = 3473, 1682, 1253, 837 cm⁻¹.
E) Eine auf 0 °C gekühlte Lösung von 0,42 g des vorstehend erhaltenen, am Stickstoff geschützten Bicyclus in 6 ml THF wurde mit 0,25 g Tetrabutylammoniumfluorid versetzt, nach 15 Minuten auf Raumtemperatur erwärmt und dann für weitere 12 Stunden gerührt. Die Reaktionsmischung wurde auf 10 ml Wasser, welches mit 5 ml Ether überschichtet war, gegossen. Nach dem Abtrennen der organischen Phase extrahierte man die wäßrige Phase dreimal mit Ether, trocknete die vereinigten organischen Phasen über Natriumsulfat und dampfte das Lösungsmittel unter vermindertem Druck ein. Chromatographie an Kieselgel (Laufmittel: Essigester/n-Hexan = 1:1 v/v) lieferte 0,35 g (-)-(2S,3S,4S,5S)-*R*_{*S*}-*N*(1*R*)-*N*-[1-(hydroxymethyl)-2-methylpropyl]-3-hydroxy-2-phenyl-5-(4-methylphenylsulfonimidoylmethyl-2-aza-(N-tert.-butoxycarbonyl)-bicyclo[3.3.0]octan. [α]_{D}²⁰ = -14,1° (c = 2,7 in Dichlormethan); IR(Film) = 3473, 1681, 1252 cm⁻¹.
F) Zu einer auf 0 °C gekühlten Suspension von 0,56 g Samarium in 13 ml THF wurden tropfenweise insgesamt 0,84 g Diiodmethan zugegeben. Nach erfolgter Zugabe rührte man 15 Minuten lang bei der angegeben Temperatur, bevor die Reaktionsmischung auf Raumtemperatur aufgetaut wurde. Man ließ weitere 60 Minuten lang rühren und gab dann eine Lösung von 0,28 g der vorstehend erhaltenen N-BOC-5-Sulfonimidoyl-Verbindung in einer Mischung aus 1 ml Methanol und 2 ml THF zu. Die Reaktionsmischung wurde vier Stunden lang gerührt und anschließend auf 110 ml einer gesättigten Ammoniumchloridlösung gegeben. Nach dem Abtrennen der organischen Phase gab man zu der wäßrigen Phase so lange 0,5 N Salzsäurelösung hinzu, bis die Suspension aufgeklart war. Die klare wäßrige Phase wurde zweimal mit Ether extrahiert, die vereinigten organischen Phasen über Natriumsulfat getrocknet und das Lösungsmittel unter vermindertem Druck eingedampft. Chromatographie des verbliebenen Rückstandes an Kieselgel (Laufmittel: Ether/n-Hexan = 1:4 v/v) lieferte 0,11 g der Titelverbindung als farblosen Festkörper, Fp. = 176,8 °C, [α]_{D}²⁰ = +50,7° (c = 0,56 in Dichlormethan); IR (Film) = 3439, 1661 cm⁻¹.

### Beispiel 3

### (+)-(2S,3R,4R,5S)-3-Hydroxy-5-methyl-2-phenyl-1-aza-(N-tert.-butoxycarbonyl)-bicyclo[3.3.0]octan

A) 6,3 g (-)-Sₛ-4R-Isopropyl-2-p-tolyl-4,5-dihydro-[1,2λ⁶,3]oxathiazol-2-oxid wurden mit 6,03 g tert.-Butyldimethylsilylchlorid entsprechend der in Beispiel 2A) beschriebenen Weise umgesetzt. Man erhielt 8,7 g (+)-Sₛ-N(1R)-N-[1-((tert.-Butyldimethylsilyl)oxy)methyl-2-methylpropyl]-S-methyl-S-(4-methylphenyl)sulfoximid als farbloses Öl, optischer Drehwert [α]_{D}²⁰= +89,9° (c = 1,0 in Dichlormethan), IR (Film): 1251, 1134 cm⁻¹.
B) Auf die in vorstehend unter 2B) beschriebene Weise wurde eine Lösung von 8,04 g des vorstehend erhaltenen Methylsulfoximids in 65 ml THF mit 16,3 ml einer 1,6 molaren Lösung von n-Butyllithium in n-Hexan 3,1 ml Cyclopentanon, 9,83 ml Trimethylsilyltrifluormethansulfonat und weiteren 27,19 ml einer 1,6 molaren Lösung von n-Butyllithium in n-Hexan umgesetzt. Chromatographie an Kieselgel (Laufmittel: Ether/n-Hexan = 1:6 v/v) lieferte 7,057 g (+)Sₛ-*N*((1R)-*N*-[1-((tert.-Butyldimethylsilyl)oxy)methyl-2-methylpropyl]-S-cyclopent-1-en-1-ylmethyl)-S-(4-methylphenyl)sulfoximid als farbloses Öl, optischer Drehwert [α]_{D}²⁰= +54,7° (c = 1,35 in Dichlormethan), IR = 1251, 1131-1.
C) Auf die in vorstehend unter 1E) beschriebene Weise wurde eine Lösung von 3,17 g des vorstehend erhaltenen Cyclopentenylsulfoximids in 22 ml Toluol mit 5,6 ml einer 1,6-molaren Lösung von n-Butyllithium in n-Hexan, 11,2 ml einer 1-molaren Lösung von Chlortris(isopropoxy)titan in n-Hexan, einer Lösung von 4,0 g FMOC-geschütztem S-α-Aminophenylethanol in 20 ml THF und 7,4 ml Piperidin umgesetzt. Chromatographie an Kieselgel (Laufmittel: Ether/n-Hexan = 1:1 v/v) lieferte 2,4 g (2S, 3R, 4R, 5S)-Sₛ-*N*(1R)-*N*-[1-((tert.-Butyldimethylsilyl)oxy)methyl-2-methylpropyl]-3-hydroxy-2-phenyl-5(4-methylphenylsulfonimidoylmethyl-2-azabicyclo[3.3.0]octan.
D) Zu einer Lösung von 1,58 g des vorstehend erhaltenen Bicyclus in 17 ml Dioxan und 4 ml Wasser wurden 0,35 g Natriumhydrogencarbonat und 1,21 g Di-tert.-butyldicarbonat zugegeben und 12 Stunden gerührt. Nach Eindampfen des Lösungsmittels unter vermindertem Druck wurde der erhaltene Rückstand zwischen 5 ml Wasser und 10 ml Ether verteilt. Die organische Phase wurde abgetrennt und die wäßrige Phase zweimal mit Ether extrahiert. Trocknung der vereinigten organischen Phasen über Natriumsulfat, Eindampfen des Lösungsmittels unter vermindertem Druck und Chromatographie des verbleibenden Rückstandes an Kieselgel (Laufmittel: Ether/n-Hexan 1:1 v/v) lieferte 1,52 g (+)-(2S,3R,4R,5S)-Sₛ-*N*(1R)-*N*-[1-((tert.-Butyldimethylsilyl)oxy)methyl-2-methylpropyl]-3-hydroxy-2-phenyl-5-(4-methylphenylsulfonimidoylmethyl-(2-aza-N-tert.-butoxycarbonyl)-bicyclo[3.3.0]octan, optischer Drehwert [α]_{D}²⁰= +63,2° (c = 1,0 in Dichlormethan); IR (Film) = 3473, 1694, 1254, 836 cm⁻¹.
E) Eine auf 0 °C gekühlte Lösung von 1,52 g des vorstehend erhaltenen, am Stickstoff geschützten Bicyclus in 14 ml THF wurde mit 1,43 g Tetrabutylammoniumfluorid versetzt, nach 15 Minuten auf Raumtemperatur erwärmt und dann für weitere 12 Stunden gerührt. Die Reaktionsmischung wurde auf 30 ml Wasser, welches mit 20 ml Ether überschichtet war, gegossen. Nach Abtrennung der organischen Phase extrahierte man die wäßrige Phase dreimal mit Ether, trocknete die organische Phase über Natriumsulfat und dampfte das Lösungsmittel unter vermindertem Druck ein. Chromatographie an Kieselgel (Laufmittel: Essigester/n-Hexan = 1:3 v/v) lieferte 0,96 g (+)-(2S,3R,4R,5S)-S_{S}-*N*(1R)-*N*-[2-hydroxymethyl-2-methylpropyl]-3-hydroxy-2-phenyl-5-(4-methylphenylsulfonimidoylmethyl-(2-aza-N-tert.-butoxycarbonyl)-bicyclo[3.3.0]octan, optischer Drehwert [α]_{D}²⁰= +54,3° (c = 1,03 in Dichlormethan; IR (Film) = 3446, 1690; 1239 cm⁻¹.
F) Zu einer Suspension von 2,04 g Samarium in 95 ml THF gab man bei Raumtemperatur 3,4 g Diiodmethan und ließ 60 Minuten rühren. Dann gab man eine Lösung von 0,955 g der vorstehend erhaltenen 5-Sulfonimidoyl-Verbindung in einer Mischung aus 1,7 ml Methanol und 3,4 ml THF zu. Die Reaktionsmischung wurde 16 Stunden lang gerührt und anschließend auf 100 ml Wasser gegeben. Zu dem Gemisch gab man so lange 0,5 N Salzsäurelösung hinzu, bis die Suspension aufgeklart war. Die Phasen wurden getrennt und die wäßrige Phase wurde zweimal mit Ether extrahiert, die vereinigten organischen Phasen über Natriumsulfat getrocknet und das Lösungsmittel unter vermindertem Druck eingedampft. Chromatographie des verbleibenden Rückstandes an Kieselgel (Laufmittel: Ether/n-Hexan = 1:3 v/v) lieferte 0,43 g der Titelverbindung als farbloses, erstarrendes Öl (Schaum), optischer Drehwert [α]_{D}²⁰= +34,5° (c = 1,01 in Dichlormethan; IR (Film) = 3447, 1669 cm⁻¹.

### Beispiel 4

### (-)-(2S,3R,4R,5S)-3-Hydroxy-5-methyl-2-phenyl-1-azabicyclo-[3.3.0]octan

205 mg (+)-(2S,3R,4R,5S)-3-Hydroxy-5-methyl-2-phenyl-1-aza-(N-tert.-butoxycarbonyl)-bicyclo[3.3.0]octan (Herstellung siehe Beispiel 3) wurden unter Argonatmosphäre und Feuchtigkeitsausschluß in einem Gemisch, bestehend aus 1,61 ml einer 4,0 M Chlortrimethylsilan-Lösung in Dichlormethan und 4,84 ml einer 4,0 M Phenol-Lösung in Dichlormethan, gelöst und 20 Minuten lang bei Raumtemperatur gerührt. Anschließend wurde auf 10 ml einer 10%igen wäßrigen Natronlauge gegossen, die organische Phase abgetrennt, die wäßrige Phase noch 2 mal mit jeweils 5 ml Dichlormethan und einmal mit 5 ml Ether extrahiert und die vereinigten organischen Phasen wurden über Magnesiumsulfat getrocknet. Das Lösungsmittel wurde unter vermindertem Druck eingedampft und der Rückstand wurde über Kieselgel gereinigt (Laufmittel: Essigester/n-Hexan 10:1 v/v). Man erhielt 113 mg kristalline Titelverbindung, Fp. = 84,5 °C, optischer Drehwert [α]_{D}²⁰ = -46,4° (c = 1,04 in Dichlormethan).

### Beispiel 5

### (+)-(2S,3S,4R,5S)-3-Amino-5-methyl-2-phenyl-1-aza-(N-tert.-butoxycarbonyl)-bicyclo[3.3.0]octan

A) Zu einer Lösung von 200 mg (-)-(2S,3R,4R,5S)-3-Hydroxy-5-methyl-2-phenyl-1-azabicyclo[3.3.0]octan in 1,5 ml THF wurden bei Raumtemperatur unter Argonatmosphäre und Feuchtigkeitsausschluß 241 mg Triphenylphosphin und 135 mg Phthalimid gegeben. Anschließend gab man innerhalb von 2 min. 0,14 ml DEAD zu. Nach 10 Stunden Reaktionszeit dampfte man das Lösungsmittel unter vermindertem Druck ein und nahm den Rückstand in 5 ml Ether auf. Nach Abfiltrieren von ungelöstem Rückstand und Eindampfen des Lösungsmittels unter vermindertem Druck erhielt man das (2S,3S,4R,5S)-5-Methyl-2-phenyl-3-phthalimido-1-azabicyclo[3.3.0]octan als Rohprodukt, welches ohne weitere Reinigung für die nachfolgende Reaktion verwendet wurde.
B) 174 mg des vorstehend erhaltenen Rohproduktes wurden in 3 ml Dioxan gelöst. Zu dieser Vorlage gab man 220 mg Ditert.-butyldicarbonat und 63 mg Natriumhydrogencarbonat sowie 0,5 ml Wasser zu und rührte das entstandene Gemisch 16 Stunden lang bei Raumtemperatur. Das Lösungsmittel wurde unter vermindertem Druck eingedampft und der verbliebene Rückstand wurde in Wasser und Ether aufgenommen. Man trennte die Phasen und extrahierte die wäßrige Phase 2 mal mit jeweils 5 ml Ether. Die vereinigten organischen Phasen wurden über Magnesiumsulfat getrocknet, bevor das Lösungsmittel unter vermindertem Druck eingedampft wurde. Chromatographie des verbliebenen Rückstandes an Kieselgel (Laufmittel: Ether/n-Hexan 1:3 v/v) lieferte 115 mg öliges (2S,3S,4R,5S)-5-Methyl-2-phenyl-3-phthalimido-1-aza-(N-tert.-butoxycarbonyl)-bicyclo[3.3.0]octan.
C) Eine Lösung von 115 mg des vorstehend erhaltenen Phthalimido-bicyclo[3.3.0]octans in 2 ml Ethanol wurde mit 400 mg Hydrazinhydrat (24%ig) versetzt und das entstandene Gemisch wurde 8 Stunden lang zum Rückfluß erhitzt. Man dampfte das Lösungsmittel unter vermindertem Druck ein, nahm den verbliebenen Rückstand in 10 ml Ether auf und extrahierte die organische Phase mit 10 ml einer 10%igen wäßrigen Natronlauge. Die wäßrige Phase wurde 2 mal mit jeweils 10 ml Ether extrahiert und die vereinigten organischen Phasen wurden über Magnesiumsulfat getrocknet. Man dampfte das Lösungsmittel unter vermindertem Druck ab und erhielt 74 mg kristalline Titelverbindung, Fp. = 92,1 °C, [α]_{D}²⁰ = +24,1° (c = 1,0 in Dichlormethan).

Nach den vorstehend angegebenen Methoden können auch die in der nachfolgenden Tabelle angegebenen Verbindungen der Formel I hergestellt werden.

In der Tabelle werden folgende Abkürzungen verwendet:
- i-Bu =: Isobutyl
- Bn =: Benzyl
- BOC =: tert.-Butyloxycarbonyl
- TBOM =: tert.-Butyloxymethyl
- Ph =: Phenyl
- Z. =: Zersetzung beim Erhitzen
- N.N. =: Eintrag nicht besetzt

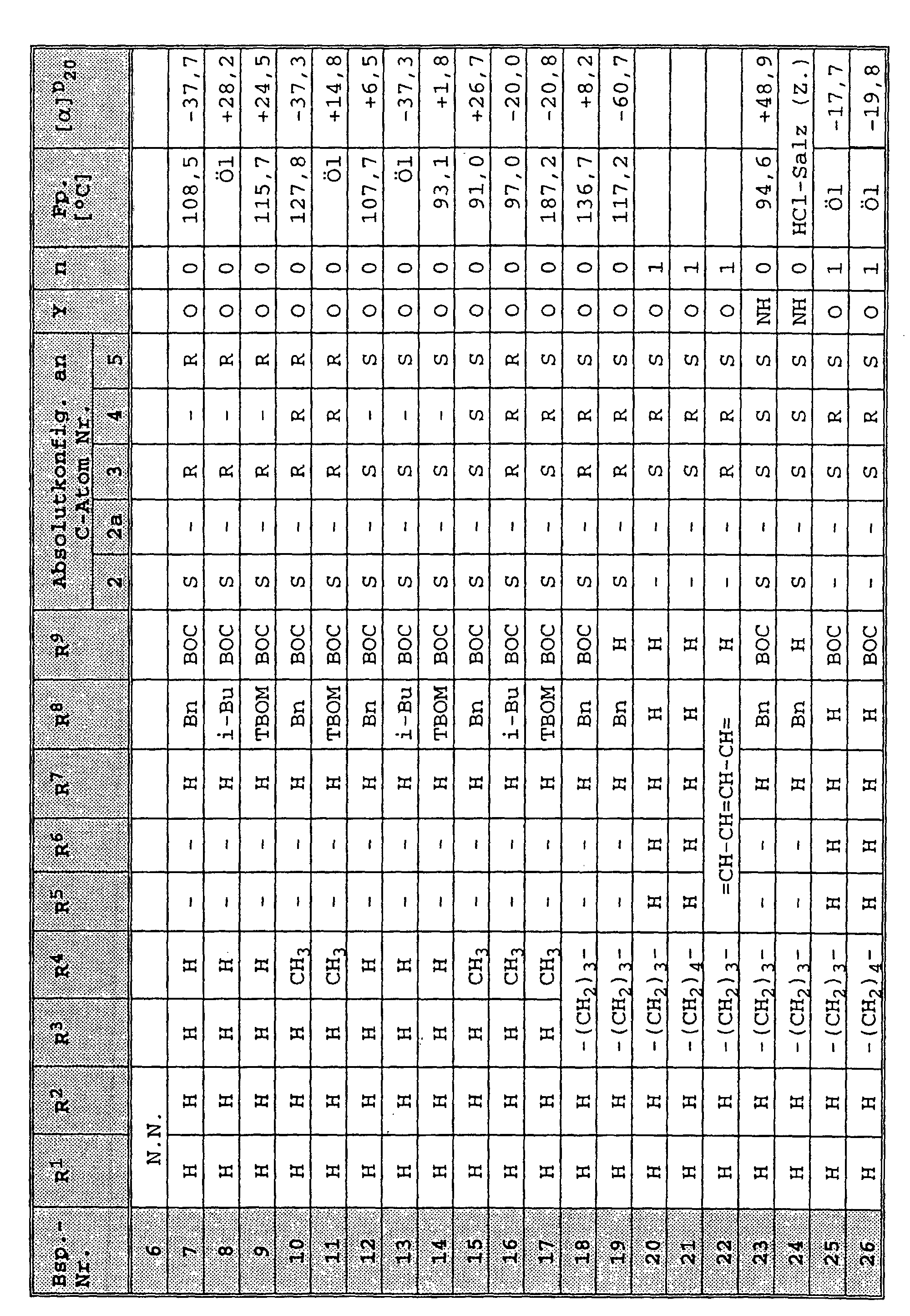

## Patentansprüche

1. Verfahren zur stereochemisch kontrollierten Herstellung von Verbindungen der allgemeinen Formel Ia', 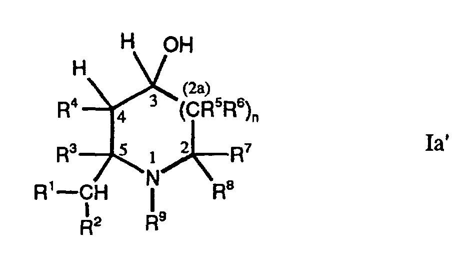 worin die R¹R²CH-Gruppe in 5-Position des cyclischen Grundgerüstes und die Hydroxy-Gruppe in 3-Position des cyclischen Grundgerüstes jeweils in trans-Stellung zueinander stehen und worin der Substituent R⁴ in 4-Position und die Hydroxy-Gruppe in 3-Position des cyclischen Grundgerüstes jeweils in cis-Stellung zueinander stehen und worin
n 0 oder 1 bedeutet,
R¹ Wasserstoff, C₁-C₆-Alkyl oder gegebenenfalls im Phenylring ein- bis dreifach durch niederes Alkyl, niederes Haloalkyl, niederes Alkoxy oder niederes Haloalkoxy substituiertes Phenyl-C₁-C₆-alkyl bedeutet und
R² Wasserstoff bedeutet,
R³ Wasserstoff bedeutet und
R⁴ Wasserstoff, niederes Alkyl oder gegebenenfalls im Phenylring ein- oder mehrfach durch niederes Alkyl, niederes Haloalkyl, niederes Alkoxy oder niederes Haloalkoxy substituiertes Phenylniederalkyl bedeutet, oder
R³ und R⁴ auch gemeinsam eine C₂-Alkylenkette oder eine gegebenenfalls 1 bis 3 Doppelbindungen enthaltende C₃-C₆-Alkylenkette bedeutet, welche durch gegebenenfalls ein- oder zweifach durch niederes Alkyl substituiertes C₁-C₂-Alkylen überbrückt sein kann,
R⁵ Wasserstoff, niederes Alkyl, Hydroxy, niederes Alkoxy oder jeweils, im Phenylring gegebenenfalls ein- bis dreifach durch niederes Alkyl, niederes Haloalkyl, niederes Alkoxy oder niederes Haloalkoxy substituiertes Phenylniederalkyl oder Phenylniederalkoxy bedeutet und
R⁶ Wasserstoff bedeutet und
R⁷ Wasserstoff bedeutet und
R⁸ Wasserstoff, Cyano, gegebenenfalls mit gegebenenfalls ein bis drei Doppelbindungen enthaltenden cycloaliphatischen oder geradkettigen oder verzweigten aliphatischen C₁-C₆-Alkoholen, welche gegebenenfalls ein- bis dreifach durch Halogen oder niederes Alkoxy substituiert sind, oder auch mit gegebenenfalls im Phenylring ein- bis dreifach durch niederes Alkyl, niederes Haloalkyl, niederes Alkoxy oder niederes Haloalkoxy substituierten Phenylniederalkoholen verestertes Carboxy, gegebenenfalls am Stickstoff einfach durch C₃-C₈-Cycloalkylniederalkanoyl oder geradkettiges oder verzweigtes aliphatisches C₁-C₆-Alkanoyl, welche jeweils gegebenenfalls ein- bis dreifach durch Halogen oder niederes Alkoxy substituiert sind, oder durch gegebenenfalls im Phenylring ein- bis dreifach durch niederes Alkyl, niederes Haloalkyl, niederes Alkoxy oder niederes Haloalkoxy substituiertes Phenylniederalkanoyl, oder am Stickstoff ein- oder zweifach durch C₃-C₈-Cycloalkylniederalkyl oder geradkettiges oder verzweigtes aliphatisches C₁-C₆-Alkyl, welche jeweils gegebenenfalls ein- bis dreifach durch Halogen oder niederes Alkoxy substituiert sind, oder durch gegebenenfalls im Phenylring ein- bis dreifach durch niederes Alkyl, niederes Haloalkyl, niederes Alkoxy oder niederes Haloalkoxy substituiertes Phenylniederalkyl, oder auch am Stickstoff mit einer geeigneten Aminoschutzgruppe substituiertes Carbonylamino, ein gegebenenfalls ein- bis vierfach ungesättigtes mono- oder bicyclisches Ringsystem mit 3 bis 10 Ringkohlenstoffatomen, dessen Ringkohlenstoffatome ein- bis dreifach durch Stickstoff, Sauerstoff und/oder Schwefel ersetzt sein können und welches Ringsystem ein- bis dreifach durch niederes Alkyl, niederes
Haloalkyl, niederes Alkoxy, Hydroxy, Halogen oder durch eine niedere Alkylenkette, welche an zwei an benachbarte Kohlenstoffatome des Ringsystems gebundene Sauerstoffatome gebunden ist, substituiert sein kann, bedeutet, oder auch für gegebenenfalls eine bis drei Doppelbindungen enthaltendes geradkettiges oder verzweigtes C₁-C₁₂-Alkyl stehen kann, welches ein- bis dreifach durch Halogen, Hydroxy, niederes Alkoxy, gegebenenfalls mit gegebenenfalls ein bis drei Doppelbindungen enthaltenden cycloaliphatischen oder geradkettigen oder verzweigten aliphatischen C₁-C₆-Alkoholen, welche gegebenenfalls ein- bis dreifach durch Halogen oder niederes Alkoxy substituiert sind, oder auch mit gegebenenfalls im Phenylring ein- bis dreifach durch niederes Alkyl, niederes Haloalkyl, niederes Alkoxy oder niederes Haloalkoxy substituierten Phenylniederalkoholen verestertes Carboxy, Cyano, Mercapto, niederes Alkylthio, Amino, niederes Alkylamino, gegebenenfalls am Stickstoff einfach durch C₃-C₈-Cycloalkylniederalkanoyl oder geradkettiges oder verzweigtes aliphatisches C₁-C₆-Alkanoyl, welche jeweils gegebenenfalls ein- bis dreifach durch Halogen oder niederes Alkoxy substituiert sind, oder durch gegebenenfalls im Phenylring ein- bis dreifach durch niederes Alkyl, niederes Haloalkyl, niederes Alkoxy oder niederes Haloalkoxy substituiertes Phenylniederalkanoyl, oder am Stickstoff ein- oder zweifach durch C₃-C₈-Cycloalkylniederalkyl oder geradkettiges oder verzweigtes aliphatisches C₁-C₆-Alkyl, welche jeweils gegebenenfalls ein- bis dreifach durch Halogen oder niederes Alkoxy substituiert sind, oder durch gegebenenfalls im Phenylring ein- bis dreifach durch niederes Alkyl, niederes Haloalkyl, niederes Alkoxy oder niederes Haloalkoxy substituiertes Phenylniederalkyl, oder auch am Stickstoff mit einer geeigneten Aminoschutzgruppe substituiertes Carbonylamino, ein gegebenenfalls ein- bis vierfach ungesättigtes mono- oder bicyclisches Ringsystem mit 3 bis 10 Ringkohlenstoffatomen, dessen Ringkohlenstoffatome einbis dreifach durch Stickstoff, Sauerstoff und/oder Schwefel ersetzt sein können und welches Ringsystem ein- bis dreifach durch niederes Alkyl, niederes Haloalkyl, niederes Alkoxy, Hydroxy, Halogen oder durch eine niedere Alkylenkette, welche an zwei an benachbarte Kohlenstoffatome des Ringsystems gebundene Sauerstoffatome gebunden ist, substituiert sein kann, oder
R⁵ und R⁸ auch gemeinsam mit den Kohlenstoffatomen, an welche sie gebunden sind, ein gegebenenfalls 1 bis 3 Doppelbindungen enthaltendes mono- oder bicyclisches Ringsystem mit 5 bis 10 Ringkohlenstoffatomen bilden können, dessen nicht die Substituenten R⁵ oder R⁸ tragende Kohlenstoffatome ein- bis dreifach durch Schwefel, Sauerstoff und/oder Stickstoff ersetzt sein können, und welches gegebenenfalls ein- bis dreifach durch niederes Alkyl, niederes Haloalkyl, niederes Alkoxy, niederes Haloalkoxy, Hydroxy, Halogen oder durch eine niedere Alkylenkette, welche an zwei an benachbarte Kohlenstoffatome des Ringsystems gebundene Sauerstoffatome gebunden ist, substituiert sein kann, oder
R⁶ und R⁷ auch gemeinsam eine Bindung bilden können und
R⁵ und R⁸ gemeinsam mit den Kohlenstoffatomen, an welche sie gebunden sind, ein aromatisches C₆-Ringsystem bilden können, welches mit 2 bis 4 weiteren Kohlenstoffatomen zu einem insgesamt 8 bis 10 Ringkohlenstoffatome enthaltenden bicyclischen Ringsystem mit insgesamt 3 bis 5 Doppelbindungen anelliert sein kann, wobei die nicht die Substituenten R⁵ oder R⁸ tragenden Kohlenstoffatome dieses C₆-C₁₀-Ringsystems ein- bis dreifach durch Schwefel, Sauerstoff und/oder Stickstoff ersetzt sein können und wobei dieses C₆-C₁₀-Ringsystem gegebenenfalls ein- bis dreifach durch niederes Alkyl, niederes Haloalkyl, niederes Alkoxy, niederes Haloalkoxy, Hydroxy, Halogen oder durch eine niedere Alkylenkette, welche an zwei an benachbarte Kohlenstoffatome des Ringsystems gebundene Sauerstoffatome gebunden ist, substituiert sein kann, und
R⁹ Wasserstoff, niederes Alkyl, gegebenenfalls im Phenylring ein- bis dreifach durch niederes Alkyl, niederes Haloalkyl, niederes Alkoxy oder niederes Haloalkoxy substituiertes Phenylniederalkyl oder eine Aminoschutzgruppe bedeutet, oder
R⁸ und R⁹ auch gemeinsam eine C₃-C₄-Alkylenkette bilden können
und deren Säureadditionssalzen, wobei gegebenenfalls vorhandene reaktive Gruppen in Verbindungen der Formel Ia' durch geeignete Schutzgruppen blockiert sein können, **dadurch gekennzeichnet, daß** man
a) eine Verbindung der allgemeinen Formel II, 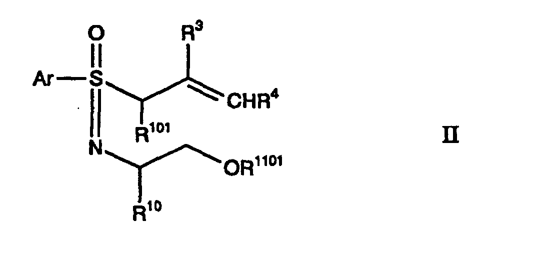 worin R³ und R⁴ obige Bedeutungen besitzen, R¹⁰¹ die vorstehend für R¹ angegebene Bedeutung besitzt, Ar für gegebenenfalls ein- bis dreifach durch niederes Alkyl substituiertes Phenyl steht, R¹⁰ niederes Alkyl oder gegebenenfalls im Phenylring einfach durch niederes Alkyl oder durch mit einer geeigneten Schutzgruppe geschütztes Hydroxy substituiertes Phenyl oder gegebenenfalls im Phenylring einfach durch niederes Alkyl substituiertes Phenylniederalkyl bedeutet und R¹¹⁰¹ für eine Silyl-Schutzgruppe steht, in einem unter den Reaktionsbedingungen inerten polaren aprotischen Lösungsmittel nacheinander zuerst bei einer Temperatur zwischen -100°C und -50 °C mit 1,05 bis 1,20 Mol, bezogen auf die Menge der eingesetzten Verbindung der Formel II einer 1 lithiierten Niederalkylverbindung umsetzt, dann die deprotonierte Form der Verbindung der Formel II bei einer Temperatur zwischen - 20 °C und 10 °C mit einem metallorganischen Reagenz der allgemeinen Formel VII,
**XM**^{**2**}**(OR**^{**12**}**)**_{**3**} **VII**
worin X für Halogen steht, M² ein vierwertiges Übergangsmetall bedeutet und R¹² für niederes Alkyl, Phenyl oder Phenylniederalkyl steht, und anschließend das erhaltene Zwischenprodukt bei Temperaturen zwischen -100°C und -50 °C mit einem Stereoisomer einer Verbindung der allgemeinen Formel VIII, 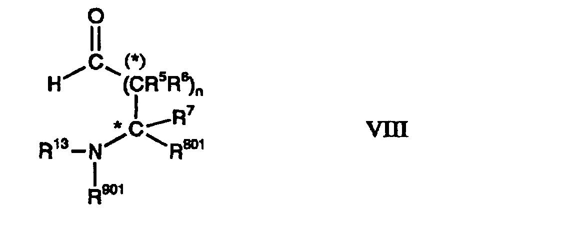 worin R⁵, R⁶, R⁷ und n die obigen Bedeutungen besitzen, R⁸⁰¹ die Bedeutung von R⁸ besitzt, wobei allfällige reaktive Gruppen nötigenfalls durch basenstabile Schutzgruppen blockiert sind, R⁹⁰¹ für Wasserstoff oder gemeinsam mit R⁸⁰¹ für eine C₃-C₄-Alkylenkette steht und R¹³ eine Amino-Schutzgruppe bedeutet, welche bei ihrer Abspaltung ein Stickstoff-Nucleophil hinterläßt, umsetzt zu einem Stereoisomer einer Verbindung der allgemeinen Formel IX, 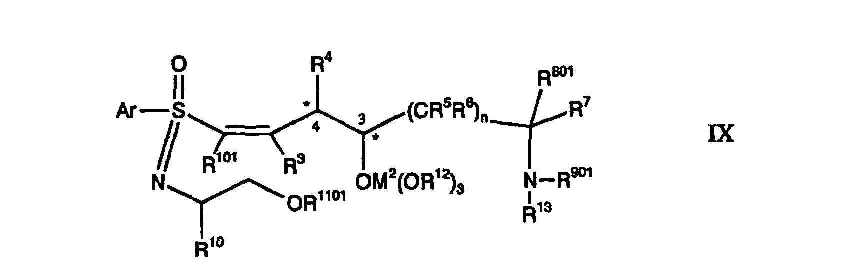 worin R¹⁰¹, R³, R⁴, R⁵, R⁶, R⁷, R⁸⁰¹, R⁹⁰¹, R¹⁰, R¹¹⁰¹, R¹², R¹³, n, Ar und M² obige Bedeutungen besitzen,
b) die erhaltene Verbindung der Formel IX bei Temperaturen zwischen -100°C und -50 °C durch Behandlung mit einem zur Entfernung der Gruppe R¹³ geeigneten Reagenz überführt in eine Verbindung der allgemeinen Formel Xa, 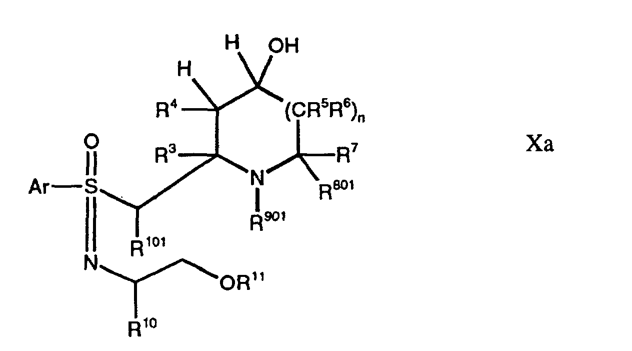 worin R¹⁰¹, R³, R⁴, R⁵, R⁶, R⁷, R⁸⁰¹, R⁹⁰¹, R¹⁰, n und Ar obige Bedeutungen besitzen und R¹¹ für Wasserstoff oder eine Silylschutzgruppe steht und, sofern R⁹⁰¹ für Wasserstoff steht, das Stickstoffatom im cyclischen Grundgerüst der entstandenen Verbindung der Formel Xa mit einer basenstabilen Schutzgruppe blockiert und eine gegebenenfalls noch vorhandene Silylschutzgruppe R¹¹ abspaltet, und
c) zur Herstellung einer Verbindung der allgemeinen Formel Ia 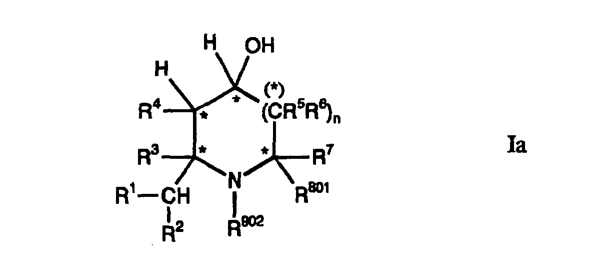 worin R¹, R², R³, R⁴, R⁵, R⁶, R⁷, R⁸⁰¹ und n obige Bedeutungen besitzen und R⁹⁰² für eine basenstabile Schutzgruppe oder gemeinsam mit R⁸⁰¹ für eine C₃-C₄-Alkylenkette steht,
eine erhaltene Verbindung der Formel Xa oder eine durch Abspaltung der Silylschutzgruppe R¹¹ entstandene Verbindung in einem polaren aprotischen Lösungsmittel bei Temperaturen zwischen -20 °C und Raumtemperatur, mit einem zur reduktiven Spaltung der Sulfonimidoyl-Alkyl-Bindung geeigneten Reduktionsmittel, ausgewählt aus der Gruppe bestehend aus Raney-Nickel, Lithiumnaphthalenid und Samarium-II-iodid, umsetzt, um eine Verbindung der allgemeinen Formel Ib, 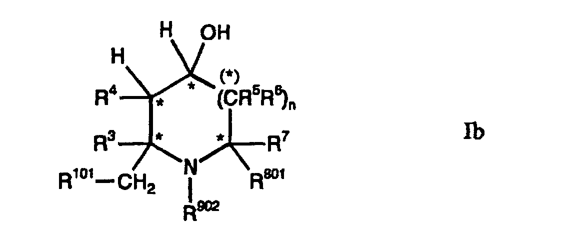 worin R¹⁰¹, R³, R⁴, R⁵, R⁶, R⁷, R⁸⁰¹, R⁹⁰² und n obige Bedeutungen besitzen, zu erhalten,
und gewünschtenfalls allfällige Schutzgruppen in Verbindungen der Formel Ia wieder abspaltet und gewünschtenfalls die gegebenenfalls freigesetzte NH-Gruppe in 1-Position des cyclischen Grundgerüstes mit einem zur N-Alkylierung oder einem zur Amidbildung befähigten Reagenz umsetzt oder mit einer Aminoschutzgruppe blockiert, um Verbindungen der Formel Ia' zu erhalten.

2. Verfahren nach Anspruch 1, worin als Aminoschutzgruppe R¹³ in Verbindungen der Formel VIII eine basenlabile Schutzgruppe verwendet wird und worin im Verfahrensschritt b) als Reagenz zur Entfernung der Schutzgruppe R¹³ eine Base verwendet wird.

3. Verfahren nach Anspruch 2, worin die basenlabile Schutzgruppe der Fluoren-9-yl-methyloxycarbonyl-Rest ist.

4. Verfahren nach Anspruch 3, worin als Base Piperidin verwendet wird.

5. Verfahren nach Anspruch 1, worin wenigstens in Verfahrensschritt a) Toluol als Lösungsmittel verwendet wird.

6. Verfahren nach Anspruch 1, worin im Verfahrensschritt c) als Reagenz für die reduktive Spaltung der Sulfonimidoyl-Alkyl Bindung in Verbindungen der allgemeinen Formel Xa Samarium-II-iodid verwendet wird.

7. Verfahren nach Anspruch 1, worin in den Verbindungen der allgemeinen Formeln Ia', Ia, Ib, II, IX und Xa jeweils R⁴ nicht Wasserstoff bedeutet.

8. Verfahren nach Anspruch 1, worin als Silylschutzgruppe R¹¹⁰¹ tert.-Butyldimethylsilyl oder Trimethylsilyl verwendet wird.

9. Verfahren nach Anspruch 1 zur Herstellung von Verbindungen der allgemeinen Formel Ia', worin R⁸ Wasserstoff, Niederalkyl, Phenyl, Phenylniederalkyl oder Niederalkoxyniederalkyl bedeutet, oder R⁶ und R⁷ gemeinsam eine Bindung bilden und R⁵ und R⁸ gemeinsam mit den Kohlenstoffatomen, an welche sie gebunden sind, ein aromatisches C₆-Ringsystem bilden oder worin R⁸ gemeinsam mit R⁹ eine C₃-C₄-Alkylenkette bildet.

10. Verbindungen der allgemeinen Formel Xa nach Anspruch 1, worin jeweils der schwefelhaltige Substituent in 5-Position und die Hydroxy-Gruppe in 3-Position des cyclischen Grundgerüstes in trans-Stellung zueinander stehen und worin der Substituent R⁴ in 4-Position und die Hydroxy-Gruppe in 3-Position des cyclischen Grundgerüstes jeweils in cis-Stellung zueinander stehen.

11. Verbindungen der allgemeinen Formel Xa nach Anspruch 10, worin R⁹⁰¹ Wasserstoff bedeutet oder gemeinsam mit R⁸⁰¹ eine C₃-C₄-Alkylenkette bildet.

12. Verwendung von Samarium-(II)-iodid zur reduktiven Desulfurierung von Alkyl-Sulfonimidoyl-Verbindungen der allgemeinen Formel Xa aus Anspruch 1.

13. Verwendung von (R_{S})-4(S)-Isopropyl-2-p-toluoyl-4,5-dihydro[1,2λ⁶,3]oxathiazol-2-oxid, (Sₛ)-4(S)-Isopropyl-2-p-toluoyl-4,5-dihydro[1,2λ⁶,3]oxathiazol-2-oxid, (Rₛ)-4(R)-Isopropyl-2-p-toluoyl-4,5-dihydro[1,2λ⁶,3]oxathiazol-2-oxid und von (S_{S})-4(R)-Isopropyl-2-p-toluoyl-4,5-dihydro[1,2λ⁶,3]-oxathiazol-2-oxid in Verfahren zur stereochemisch kontrollierten Herstellung azacyclischer Verbindungen nach Anspruch 1.

14. Verwendung von [S_{S},N(1S)]-N-[1-[[tert.-Butyldimethylsilyl)oxy]methyl]-2-methylpropyl]-S-methyl-S-(4-methylphenyl)-sulfoximid und von [R_{S},N(1R)]-N-[1-[[tert.-Butyldimethylsilyl)-oxy]methyl]-2-methylpropyl]-S-methyl-S-(4-methylphenyl)sulfoximid in Verfahren zur stereochemisch kontrollierten Herstellung azacyclischer Verbindungen nach Anspruch 1.

15. Verfahren zur Herstellung von Verbindungen der allgemeinen Formel Ib durch reduktive Desulfurierung von Verbindungen der allgemeinen Formel Xa nach Anspruch 1, **dadurch gekennzeichnet, daß** man eine Verbindung der Formel Xa in einem polaren aprotischen Lösungsmittel oder in Gemischen solcher Lösungsmittel bei Temperaturen zwischen -20 °C und Raumtemperatur und in Anwesenheit von 2 bis 5 Äquivalenten einer Protonenquelle, bezogen auf ein Äquivalent der in der eingesetzten Menge an Verbindung der Formel Xa enthaltenen Menge an Schwefel, mit Samarium-II-iodid in einem Molverhältnis von 3:1 bis 7:1, bezogen auf die eingesetzte Verbindung der Formel Xa, umsetzt.

## Claims

1. A process for the stereochemically controlled production of compounds of the general formula Ia', 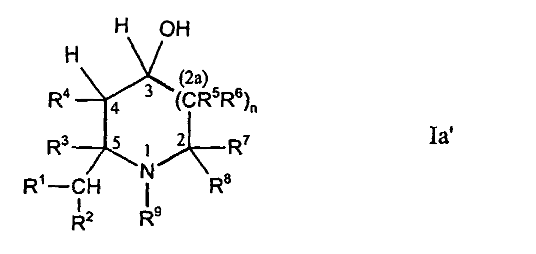 wherein the R¹R²CH group in the 5-position of the cyclic parent structure and the hydroxy group in the 3-position of the cyclic parent structure are each in the trans position relative to each other and wherein the substituent R⁴ in the 4-position and the hydroxy group in the 3-position of the cyclic parent structure are each in the cis position relative to each other, and wherein
n is 0 or 1,
R¹ is hydrogen; C₁-C₆-alkyl; or phenyl-C₁-C₆-alkyl optionally substituted one to three times in the phenyl ring by lower alkyl, lower haloalkyl, lower alkoxy or lower haloalkoxy, and
R² is hydrogen,
R³ is hydrogen, and
R⁴ is hydrogen; lower alkyl; or phenyl-lower alkyl optionally substituted one or more times in the phenyl ring by lower alkyl, lower haloalkyl, lower alkoxy or lower haloalkoxy, or
R³ and R⁴ also together are a C₂-alkylene chain; or a C₃-C₆-alkylene chain optionally containing 1 to 3 double bonds, which may be bridged by C₁-C₂-alkylene which is optionally substituted one or two times by lower alkyl,
R⁵ is hydrogen; lower alkyl; hydroxy; lower alkoxy; or phenyl-lower alkoxy or phenyl-lower alkyl each of which may be optionally substituted one to three times in the phenyl ring by lower alkyl, lower haloalkyl, lower alkoxy or lower haloalkoxy, and
R⁶ is hydrogen, and
R⁷ is hydrogen, and
R⁸ is hydrogen; cyano; carboxy optionally esterified with cycloaliphatic or straight-chain or branched aliphatic C₁-C₆-alcohols containing optionally one to three double bonds, which are optionally substituted one to three times by halogen or lower alkoxy, or alternatively with phenyl-lower alcohols optionally substituted in the phenyl ring one to three times by lower alkyl, lower haloalkyl, lower alkoxy or lower haloalkoxy, carbonylamino optionally substituted at the nitrogen once by C₃-C₈-cycloalkyl lower alkanoyl or straight-chain or branched aliphatic C₁-C₆-alkanoyl, which in each case are optionally substituted one to three times by halogen or lower alkoxy, or by phenyl-lower alkanoyl optionally substituted one to three times in the phenyl ring by lower alkyl, lower haloalkyl, lower alkoxy or lower haloalkoxy, or at the nitrogen one or two times by C₃-C₈-cycloalkyl-lower alkyl or straight-chain or branched aliphatic C₁-C₆-alkyl, which in each case are optionally substituted one to three times by halogen or lower alkoxy, or by phenyl-lower alkyl optionally substituted one to three times in the phenyl ring by lower alkyl, lower haloalkyl, lower alkoxy or lower haloalkoxy, or also carbonylamino substituted at the nitrogen with a suitable amino protecting group, a monocyclic or bicyclic ring system with 3 to 10 ring carbon atoms which is optionally unsaturated one to four times, the ring carbon atoms of which may be replaced one to three times by nitrogen, oxygen and/or sulphur and which ring system may be substituted one to three times by lower alkyl, lower haloalkyl, lower alkoxy, hydroxy, halogen or by a lower alkylene chain which is bonded to two oxygen atoms bonded to adjacent carbon atoms of the ring system, or
may also stand for straight-chain or branched C₁-C₁₂-alkyl optionally containing one to three double bonds, which may optionally be substituted one to three times by halogen, hydroxy, lower alkoxy, carboxy optionally esterified with cycloaliphatic or straight-chain or branched aliphatic C₁-C₆-alcohols, which optionally contain one to three double bonds, and which are optionally substituted one to three times by halogen or lower alkoxy, or alternatively carboxy esterified with phenyl-lower alcohols optionally substituted in the phenyl ring one to three times by lower alkyl, lower haloalkyl, lower alkoxy or lower haloalkoxy, cyano, mercapto, lower alkylthio, amino, lower alkylamino, carbonylamino optionally substituted once at the nitrogen by C₃-C₈-cycloalkyl-lower alkanoyl or straight-chain or branched aliphatic C₁-C₆-alkanoyl, which in each case are optionally substituted one to three times by halogen or lower alkoxy, or by phenyl-lower alkanoyl optionally substituted one to three times in the phenyl ring by lower alkyl, lower haloalkyl, lower alkoxy or lower haloalkoxy, or carbonylamino substituted once or twice at the nitrogen by C₃-C₈-cycloalkyl-lower alkyl or straight-chain or branched aliphatic C₁-C₆-alkyl, which are each optionally substituted one to three times by halogen or lower alkoxy, or by phenyl-lower alkyl optionally substituted one to three times in the phenyl ring by lower alkyl, lower haloalkyl, lower alkoxy or lower haloalkoxy, or alternatively carbonylamino substituted at the nitrogen with a suitable amino protecting group, a monocyclic or bicyclic ring system with 3 to 10 ring carbon atoms which is optionally unsaturated one to four times, the ring carbon atoms of which may be replaced one to three times by nitrogen, oxygen and/or sulphur and which ring system may be substituted one to three times by lower alkyl, lower haloalkyl, lower alkoxy, hydroxy, halogen or by a lower alkylene chain which is bonded to two oxygen atoms bonded to adjacent carbon atoms of the ring system, or
R⁵ and R⁸ also, together with the carbon atoms to which they are bonded, may form a monocyclic or bicyclic ring system with 5 to 10 ring carbon atoms which optionally contains 1 to 3 double bonds, the carbon atoms of which which do not bear the substituents R⁵ or R⁸ may be replaced one to three times by sulphur, oxygen and/or nitrogen, and which optionally may be substituted one to three times by lower alkyl, lower haloalkyl, lower alkoxy, lower haloalkoxy, hydroxy, halogen or by a lower alkylene chain which is bonded to two oxygen atoms bonded to adjacent carbon atoms of the ring system, or
R⁶ and R⁷ also together may form a bond, and
R⁵ and R⁸, together with the carbon atoms to which they are bonded, may form an aromatic C₆-ring system which may be fused with 2 to 4 further carbon atoms to form a bicyclic ring system having a total of 3 to 5 double bonds which contains a total of 8 to 10 ring carbon atoms, wherein the carbon atoms of this C₆- to C₁₀-ring system which do not bear the substituents R⁵ or R⁸ may be replaced one to three times by sulphur, oxygen and/or nitrogen, and wherein this C₆- to C₁₀-ring system may optionally be substituted one to three times by lower alkyl, lower haloalkyl, lower alkoxy, lower haloalkoxy, hydroxy, halogen or by a lower alkylene chain which is bonded to two oxygen atoms bonded to adjacent carbon atoms of the ring system, and
R⁹ is hydrogen; lower alkyl; phenyl-lower alkyl optionally substituted one to three times in the phenyl ring by lower alkyl, lower haloalkyl, lower alkoxy or lower haloalkoxy; or an amino protecting group, or
R⁸ and R⁹ also together may form a C₃-C₄-alkylene chain,
and their acid addition salts, wherein any reactive groups which may be present may be blocked in compounds of Formula Ia' by suitable protecting groups, **characterised in that**
a) a compound of the general formula II, 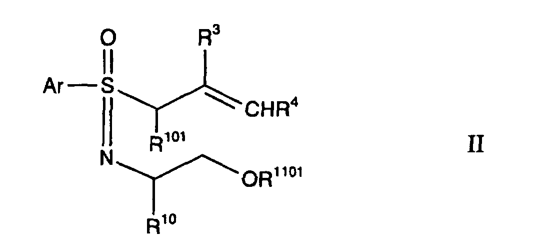 wherein R³ and R⁴ have the above meanings, R¹⁰¹ has the meaning given above for R¹, Ar stands for phenyl optionally substituted one to three times by lower alkyl, R¹⁰ is lower alkyl, or phenyl optionally substituted once in the phenyl ring by lower alkyl or by hydroxy protected with a suitable protecting group, or phenyl-lower alkyl optionally substituted once in the phenyl ring by lower alkyl, and R¹¹⁰¹ stands for a silyl protecting group,
is reacted in a polar aprotic solvent which is inert under the reaction conditions in succession first at a temperature between -100°C and -50°C with 1.05 to 1.20 mole, relative to the amount of compound of Formula II used, of a 1-lithiated lower alkyl compound, then the deprotonated form of the compound of Formula II at a temperature between -20°C and 10°C with an organometallic reagent of the general formula VII,
XM²(OR¹²)₃ VII
wherein X stands for halogen, M² is a tetravalent transition metal and R¹² stands for lower alkyl, phenyl or phenyl-lower alkyl, and then the resulting intermediate product is reacted at temperatures between -100°C and -50°C with a stereoisomer of a compound of the general formula VIII, 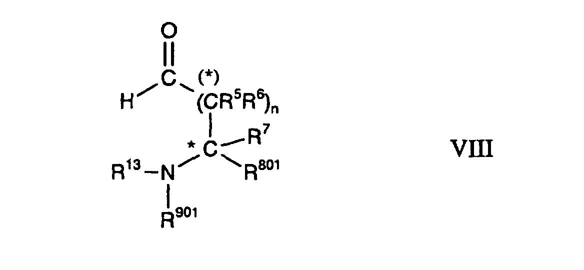 wherein R⁵, R⁶, R⁷ and n have the above meanings, R⁸⁰¹ has the meaning of R⁸, with any reactive groups if necessary being blocked by base-stable protecting groups, R⁹⁰¹ stands for hydrogen or together with R⁸⁰¹ stands for a C₃-C₄-alkylene chain and R¹³ is an amino protecting group which when cleaved leaves behind a nitrogen nucleophile, to form a stereoisomer of a compound of the general formula IX, 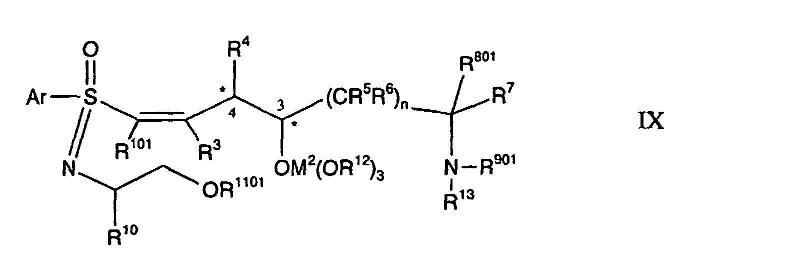 wherein R¹⁰¹, R³, R⁴, R⁵, R⁶, R⁷, R⁸⁰¹, R⁹⁰¹, R¹⁰, R¹¹⁰¹, R¹², R¹³, n, Ar and M² have the above meanings,
b) the resulting compound of Formula IX is converted at temperatures between -100°C and -50°C, by treatment with a reagent suitable for removing the group R¹³, into a compound of the general formula Xa, 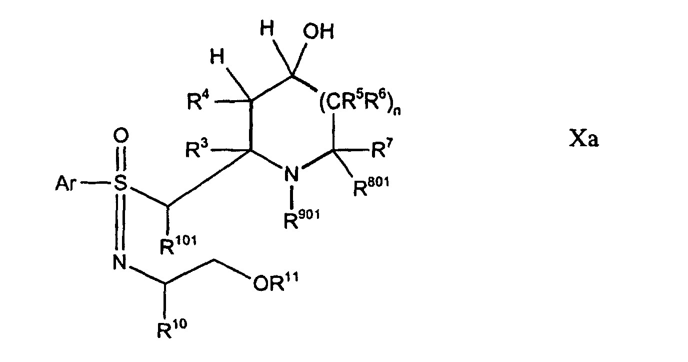 wherein R¹⁰¹, R³, R⁴, R⁵, R⁶, R⁷, R⁸⁰¹, R⁹⁰¹, R¹⁰, n and Ar have the above meanings and R¹¹ stands for hydrogen or a silyl protecting group and, if R⁹⁰¹ stands for hydrogen, the nitrogen atom in the cyclic parent structure of the resulting compound of Formula Xa is blocked with a base-stable protecting group and any silyl protecting group R¹¹ which may still be present is cleaved off, and
c) for the production of a compound of the general formula la, 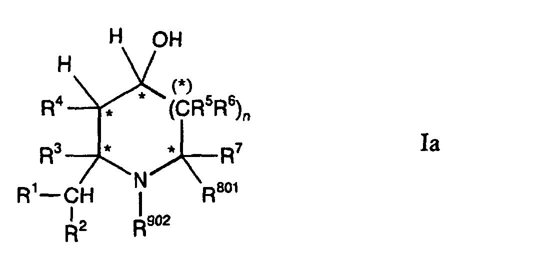 wherein R¹, R², R³, R⁴, R⁵, R⁶, R⁷, R⁸⁰¹ and n have the above meanings and R⁹⁰² stands for a base-stable protecting group or, together with R⁸⁰¹, for a C₃-C₄-alkylene chain,
a resulting compound of Formula Xa or a compound produced by cleaving off the silyl protecting group R¹¹ is reacted in a polar aprotic solvent at temperatures between -20°C and room temperature with a reagent suitable for the reductive cleavage of the sulphonimidoyl-alkyl bond, selected from the group consisting of Raney nickel, lithium naphthalenide and samarium (II) iodide, in order to obtain a compound of the general formula Ib, 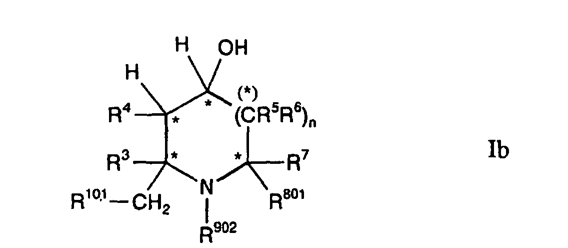 wherein R¹⁰¹, R³, R⁴, R⁵, R⁶, R⁷, R⁸⁰¹, R⁹⁰² and n have the above meanings,
and if desired any protecting groups are cleaved again in compounds of Formula la and if desired the optionally released NH group in the 1-position of the cyclic parent structure is reacted with a reagent capable of N-alkylation or one capable of amide formation or is blocked with an amino protecting group, in order to obtain compounds of Formula Ia'.

2. A process according to Claim 1, wherein a base-labile protecting group is used as the amino protecting group R¹³ in compounds of Formula VIII and wherein in process step b) a base is used as the reagent for removing the protecting group R¹³.

3. A process according to Claim 2, wherein the base-labile protecting group is the fluoren-9-yl-methyloxy-carbonyl radical.

4. A process according to Claim 3, wherein piperidine is used as the base.

5. A process according to Claim 1, wherein toluene is used as solvent at least in process step a).

6. A process according to Claim 1, wherein samarium (II) iodide is used as reagent for the reductive cleavage of the sulphonimidoyl-alkyl bond in compounds of the general formula Xa in process step c).

7. A process according to Claim 1, wherein R⁴ is not hydrogen in each of the compounds of the general formulae Ia', la, Ib, II, IX and Xa.

8. A process according to Claim 1, wherein tert. butyldimethylsilyl or trimethylsilyl is used as the silyl protecting group R¹¹⁰¹.

9. A process according to Claim 1 for the production of compounds of the general formula Ia', wherein R⁸ is hydrogen, lower alkyl, phenyl, phenyl-lower alkyl or lower-alkoxy lower alkyl, or R⁶ and R⁷ together form a bond and R⁵ and R⁸, together with the carbon atoms to which they are bonded, form an aromatic C₆-ring system or wherein R⁸ together with R⁹ forms a C₃-C₄-alkylene chain.

10. Compounds of the general formula Xa according to Claim 1, wherein in each case the sulphur-containing substituent in the 5-position and the hydroxy group in the 3-position of the cyclic parent structure are in the trans position relative to each other and wherein the substituent R⁴ in the 4-position and the hydroxy group in the 3-position of the cyclic parent structure are each in the cis position relative to each other.

11. Compounds of the general formula Xa according to Claim 10, wherein R⁹⁰¹ is hydrogen or together with R⁸⁰¹ forms a C₃-C₄-alkylene chain.

12. The use of samarium (II) iodide for the reductive desulphurisation of alkyl-sulphonimidoyl compounds of the general formula Xa from Claim 1.

13. The use of (R_{S})-4(S)-isopropyl-2-p-toluoyl-4,5-dihydro[1,2λ⁶,3]-oxathiazol-2-oxide, (Sₛ)-4(S)-isopropyl-2-p-toluoyl-4,5-dihydro[1,2λ⁶,3]-oxathiazol-2-oxide, (Rₛ)-4(R)-isopropyl-2-p-toluoyl-4,5-dihydro[1,2λ⁶,3]-oxathiazol-2-oxide and of (S_{S})-4(R)-isopropyl-2-p-toluoyl-4,5-dihydro[1,2λ⁶,3]-oxathiazol-2-oxide in processes for the stereochemically controlled production of azacyclic compounds according to Claim 1.

14. The use of [S_{S},N(1S)]-N-[1-[[tert.-butyldimethylsilyl)oxy]methyl]-2-methylpropyl]-S-methyl-S-(4-methylphenyl)sulphoximide and of [R_{S},N(1R)]-N-[1-[[tert.-butyldimethylsilyl)oxy]methyl]-2-methylpropyl]-S-methyl-S-(4-methylphenyl)sulphoximide in processes for the stereochemically controlled production of azacyclic compounds according to Claim 1.

15. A process for the production of compounds of the general formula Ib by reductive desulphurisation of compounds of the general formula Xa according to Claim 1, **characterised in that** a compound of formula Xa is reacted in a polar aprotic solvent or in mixtures of such solvents at temperatures between -20°C and room temperature and in the presence of 2 to 5 equivalents of a proton source, relative to one equivalent of the amount of sulphur contained in the amount of compound of formula Xa used, with samarium (II) iodide in a molar ratio of 3:1 to 7:1, relative to the compound of formula Xa used.

## Revendications

1. Procédé de préparation stéréochimiquement contrôlée de composés de formule générale Ia', 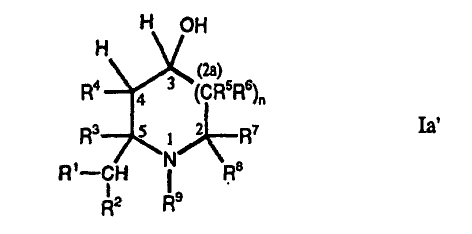 dans laquelle le groupe R¹R²CH en position 5 du squelette cyclique et le groupe hydroxy en position 3 du squelette cyclique sont respectivement en position trans l'un par rapport à l'autre, et dans laquelle le substituant R⁴ en position 4 et le groupe hydroxy en position 3 du squelette cyclique sont respectivement en position cis l'un par rapport à l'autre, et dans laquelle
n vaut 0 ou 1,
R¹ représente un hydrogène, un alkyle en C₁-C₆ ou un phényl-alkyle en C₁-C₆ éventuellement mono- à tri-substitué dans le noyau phényle par un alkyle inférieur, un haloalkyle inférieur, un alcoxy inférieur ou un haloalcoxy inférieur et
R² représente un hydrogène,
R³ représente un hydrogène, et
R⁴ représente un hydrogène, un alkyle inférieur ou un phényl-alkyle inférieur éventuellement mono ou polysubstitué dans le noyau phényle par un alkyle inférieur, un haloalkyle inférieur, un alcoxy inférieur ou un haloalcoxy inférieur, ou
R³ et R⁴ représentent également ensemble une chaîne alkylène en C₂ ou une chaîne alkylène en C₃-C₆ contenant éventuellement 1 à 3 doubles liaisons, qui peut être reliée par un alkylène en C₁-C₂, éventuellement mono- ou bi-substitué par un alkyle inférieur,
R⁵ représente un hydrogène, un alkyle inférieur, un hydroxy, un alcoxy inférieur, un phényl-alkyle inférieur ou un phényl-alcoxy inférieur éventuellement mono- à tri-substitué dans le noyau phényle par un alkyle inférieur, un haloalkyle inférieur, un alcoxy inférieur ou un haloalcoxy inférieur, et
R⁶ représente un hydrogène, et
R⁷ représente un hydrogène, et
R⁸ représente un hydrogène, un cyano, un carboxy éventuellement estérifié avec des alcools en C₁-C₆ cycloaliphatiques ou aliphatiques à chaîne linéaire ou ramifiée, contenant éventuellement une à trois doubles liaisons, qui sont éventuellement mono- à tri-substitués par un halogène ou un alkyle inférieur, ou aussi avec des phényl-alcools inférieurs mono- à tri-substitués dans le noyau phényle par un alkyle inférieur, un haloalkyle inférieur, un alcoxy inférieur ou un haloalcoxy inférieur, un carbonylamino éventuellement monosubstitué sur l'atome d'azote par un cyclo(en C₃-C₈)-alkyle inférieur-alcanoyle ou par un alcanoyle en C₁-C₆, aliphatique, à chaîne linaire ou ramifiée, qui sont éventuellement mono- à tri-substitués par un halogène ou un alcoxy inférieur, ou par un phényl-alcanoyle inférieur éventuellement mono- à tri-substitué dans le noyau phényle par un alkyle inférieur, un haloalkyle inférieur, un alcoxy inférieur ou un haloalcoxy inférieur, ou mono- ou bi-substitué sur l'atome d'azote par un cyclo(en C₃-C₈)-alkyle inférieur-alkyle ou un alkyle en C₁-C₆ aliphatique linéaire ou ramifié, qui sont éventuellement mono- à tri-substitués par un halogène ou un alcoxy inférieur, ou par un phényl-alkyle inférieur éventuellement mono- à tri-substitué dans le noyau phényle par un alkyle inférieur, un haloalkyle inférieur, un alcoxy inférieur ou un haloalcoxy inférieur, ou substitué sur l'atome d'azote par un groupe protecteur amino approprié, un système cyclique mono- ou bi-cyclique éventuellement mono- à quadri-insaturé avec 3 à 10 atomes de carbone dans le cycle, dont les atomes de carbone du cycle peuvent être mono- à ter-substitués par de l'azote, de l'oxygène et/ou du soufre, et dans lequel système cyclique peut être mono- à tri-substitué par un alkyle inférieur, un haloalkyle inférieur, un alcoxy inférieur, un hydroxy, un halogène ou par une chaîne alkylène inférieur qui est liée sur deux atomes d'oxygène liés à des atomes de carbone voisins du système cyclique, ou encore peut également représenter un alkyle en C₁-C₁₂ à chaîne linéaire ou ramifiée contenant le cas échéant une à trois doubles liaisons, lequel peut être mono- à tri-substitué par un halogène hydroxy, un alcoxy inférieur, un carboxy éventuellement estérifié avec des alcools en C₁-C₆ cycloaliphatiques ou aliphatiques à chaîne linéaire ou ramifiée, contenant éventuellement une à trois doubles liaisons, éventuellement mono- à tri-substitué par un halogène ou un alcoxy inférieur ou avec des phényl-alcools inférieurs éventuellement mono- à tri-substitué dans le noyau phényle par un alkyle inférieur, un haloalkyle inférieur, ou alcoxy inférieur ou un haloalcoxy inférieur, un cyano, un mercapto, un alkyle inférieur-thio, un amino, un alkyle inférieur-amino, un carbonylamino éventuellement monosubstitué sur l'atome d'azote par un cyclo(en C₃-C₈)-alkyle inférieur-alcanoyle ou par un alcanoyle en C₁-C₆, aliphatique, à chaîne linaire ou ramifiée, qui sont éventuellement mono- à tri-substitués par un halogène ou un alcoxy inférieur, ou par un phényl-alcanoyle inférieur éventuellement mono- à tri-substitué dans le noyau phényle par un alkyle inférieur, un haloalkyle inférieur, un alcoxy inférieur ou un haloalcoxy inférieur, ou mono- ou bi-substitué sur l'atome d'azote par un cyclo(en C₃-C₈)-alkyle inférieur-alkyle ou un alkyle en C₁-C₆ aliphatique linéaire ou ramifié, qui sont éventuellement mono- à tri-substitués par un halogène ou un alcoxy inférieur, ou par un phényl-alkyle inférieur éventuellement mono- à tri-substitué dans le noyau phényle par un alkyle inférieur, un haloalkyle inférieur, un alcoxy inférieur ou un haloalcoxy inférieur, ou substitué sur l'atome d'azote par un groupe protecteur amino approprié, un système cyclique mono- ou bi-cyclique éventuellement mono- à quadri-insaturé et ayant 3 à 10 atomes de carbone dans le cycle, dont les atomes de carbone du cycle peuvent être remplacés une à trois fois par de l'azote, de l'oxygène et/ou du soufre, et lequel système cycliques peut être mono- à tri-substitué par un alkyle inférieur, un haloalkyle inférieur, un alcoxy inférieur, un hydroxy, un halogène ou par une chaîne alkylène inférieur qui est liée à deux atomes d'oxygène liés à deux atomes de carbone voisins du système cyclique, ou
R⁵ et R⁸ peuvent également former avec l'atome de carbone auquel ils sont liés un système mono- ou bi-cyclique contenant éventuellement 1 à 3 doubles liaisons et comportant 5 à 10 atomes de carbone dans le cycle, dont les atomes de carbone ne portant pas les substituant R⁵ ou R⁸ peuvent être remplacés une à trois fois par du soufre, de l'oxygène et/ou de l'azote, et qui peut le cas échéant être mono- à tri-substitué par un alkyle inférieur, un haloalkyle inférieur, un alcoxy inférieur, un haloalcoxy inférieur un hydroxy, un halogène ou par une chaîne alkylène inférieure qui est liée à deux atomes d'oxygène liés à des atomes de carbone voisins du système cyclique, ou
R⁶ et R⁷ peuvent également former ensemble une liaison et
R⁵ et R⁸ peuvent former ensemble avec l'atome de carbone auquel ils sont liés un système cyclique en C₆ aromatique qui peut être condensé avec 2 à 4 autres atomes de carbone pour donner un système bicyclique contenant au total 8 à 10 atomes de carbone cycliques avec au total 3 à 5 doubles liaisons, les atomes de carbone ne portant pas les substituants R⁵ ou R⁸ de ce système cyclique en C₆-C₁₀ pouvant être remplacés une à trois fois par du soufre, de l'oxygène et/ou de l'azote, et ce système cyclique en C₆-C₁₀ pouvant être le cas échéant mono- à tri-substitué par un alkyle inférieur, un haloalkyle inférieur, un alcoxy inférieur, un haloalcoxy inférieur, un hydroxy, un halogène ou par une chaîne alkylène inférieur qui est liée à deux atomes d'oxygène, liés à des atomes de carbone voisins du système cyclique, et
R⁹ représente un hydrogène, un alkyle inférieur, un phényl-alkyle inférieur éventuellement mono- à tri-substitué dans le noyau phényle par un alkyle inférieur, un haloalkyle inférieur, un alcoxy inférieur, un haloalcoxy inférieur, ou un groupe protecteur d'amino, ou
R⁸ et R⁹ peuvent également former ensemble une chaîne alkylène en C₃-C₄, et leurs sels d'addition d'acides, où les groupes réactifs éventuellement présents dans les composés de formule Ia' peuvent être bloqués par des groupes protecteurs appropriés, **caractérisé en ce que**
a) on fait réagir un composé de formule générale II, 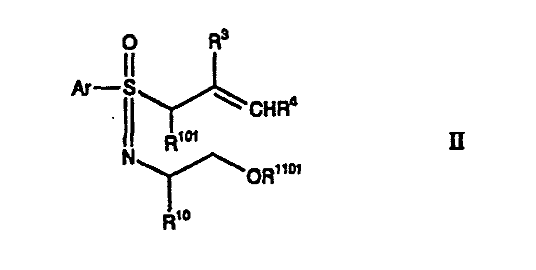 dans laquelle R³ et R⁴ ont les significations données ci-dessus, R¹⁰¹ a la signification indiquée ci-dessus pour R¹, Ar représente un phényle éventuellement mono- à tri-substitué par un alkyle inférieur, R¹⁰ représente un alkyle inférieur ou un phényle éventuellement monosubstitué dans le noyau phényle par un alkyle inférieur ou par un hydroxy protégé par un groupe protecteur approprié, ou un phényl-alkyle inférieur éventuellement monosubstitué dans le noyau phényle par un alkyle inférieur, et R¹¹⁰¹ représente un groupe protecteur de silyle dans un solvant polaire aprotique inerte dans les conditions réactionnelles successivement d'abord à une température comprise entre -100°C et -50°C, avec 1,05 à 1,20 mol, sur la base de la quantité de composé de formule II utilisée, d'un composé alkyle inférieur lithié, puis la forme déprotonée du composé de formule II, à une température comprise entre -20°C et 10°C, avec un réactif organométallique de formule générale VII,
XM²(OR¹²)₃ VII
dans laquelle X représente un halogène, M² représente un métal de transition tétravalent et R¹² représente un alkyle inférieur, un phényle ou un phényl-alkyle inférieur, puis le produit intermédiaire obtenu à des températures comprises entre -100°C et -50°C, avec un stéréoisomère d'un composé de formule générale VIII, 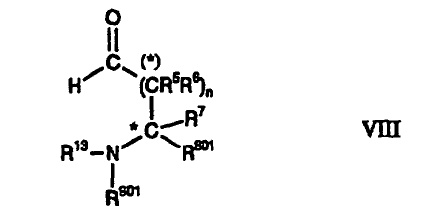 dans laquelle R⁵, R⁶, R⁷ et n ont les significations données ci-dessus, R⁸⁰¹ a la signification de R⁸ où les groupes réactifs éventuels sont bloqués si nécessaire par les groupes protecteurs stables vis-à-vis des bases, R⁹⁰¹ représente un hydrogène ou, avec R⁸⁰¹, une chaîne alkylène en C₃-C₄, et R¹³ représente un groupe protecteur d'amino qui laisse lors de sa coupure un corps nucléophile azoté, pour donner un stéréoisomère d'un composé de formule générale IX, 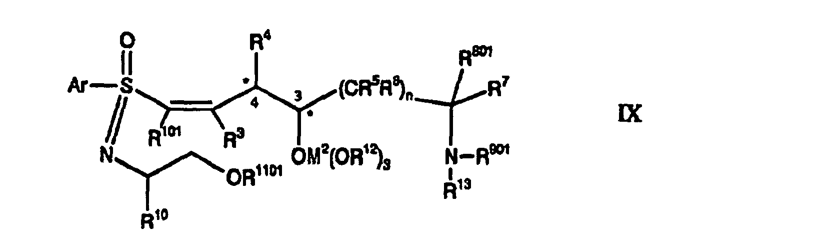 dans laquelle R¹⁰¹, R³, R⁴, R⁵, R⁶, R⁷, R⁸⁰¹, R⁹⁰¹, R¹⁰, R¹¹⁰¹, R¹², R¹³, n, Ar et M² ont les significations indiquées ci-dessus,
b) on transforme le composé de formule IX obtenu à des températures comprises entre -100°C et -50°C par un traitement avec un réactif approprié pour l'élimination du groupe R¹³, en un composé de formule générale Xa, 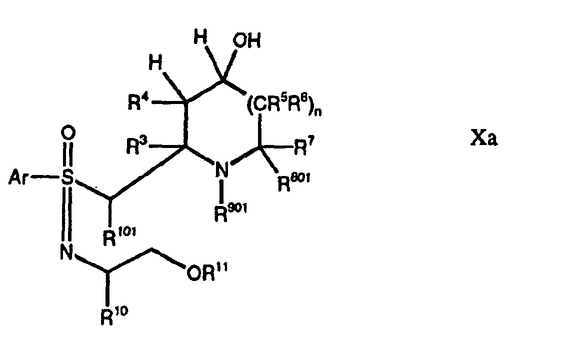 dans laquelle R¹⁰¹, R³, R⁴, R⁵, R⁶, R⁷, R ⁸⁰¹, R⁹⁰¹, R¹⁰, n et Ar ont les significations indiquées ci-dessus et R¹¹ représente un hydrogène ou un groupe protecteur de silyle et dans la mesure où R⁹⁰¹ représente un hydrogène, on bloque l'atome d'azote dans l'ossature de base cyclique du composé de formule Xa obtenu avec un groupe protecteur stable vis-à-vis des bases et on sépare un groupe protecteur de silyle R¹¹ éventuellement encore présent, et
c) pour préparer un composé de formule générale Ia, 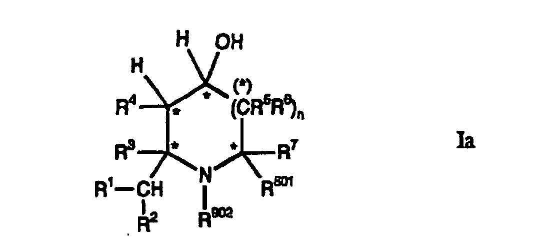
dans laquelle R¹, R², R³, R⁴, R⁵, R⁶, R⁷, R⁸⁰¹ et n ont les significations données ci-dessus et R⁹⁰² représente un groupe protecteur stable vis-à-vis des bases ou, avec R⁸⁰¹, ont une chaîne alkylène en C₃-C₄,
on fait réagir un composé obtenu de formule Xa ou un composé apparu par séparation du groupe protecteur de silyle R¹¹ dans un solvant polaire aprotique, à des températures comprises entre -20°C et la température ambiante avec un agent réducteur approprié pour le clivage par réduction de la liaison sulfonimidoyl-alkyle sélectionné parmi le groupe composé du nickel de Raney, de naphtalénure de lithium et du iodure de samarium II, pour obtenir un composé de formule générale Ib, 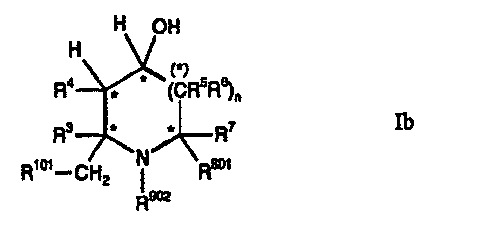 dans laquelle R¹⁰¹, R³, R⁴, R⁵, R⁶, R⁷, R⁸⁰¹, R⁹⁰² et n ont les significations indiquées ci-dessus,
et si cela est souhaité, on sépare à nouveau les groupes protecteurs présents dans les composés de formule Ia et si on le désire, on fait réagir le groupe NH éventuellement libéré en position 1 de l'ossature de base cyclique avec un réactif apte à la N-alkylation ou un réactif apte à la formation d'un amide, ou on bloque avec un groupe protecteur d'amino, pour obtenir des composés de formule Ia'.

2. Procédé selon la revendication 1, dans lequel on utilise comme groupe protecteur d'amino R¹³ dans les composés de formule VIII un groupe protecteur labile vis-à-vis des bases et dans lequel dans l'étape b) du procédé, on utilise comme réactif une base pour l'élimination du groupe protecteur R¹³.

3. Procédé selon la revendication 2, dans lequel le groupe protecteur labile vis-à-vis des bases est le radical fluorén-9-yl-méthyloxycarbonyle.

4. Procédé selon la revendication 3, dans lequel on utilise comme base la pipéridine.

5. Procédé selon la revendication 1, dans lequel on utilise au moins dans l'étape a) du procédé le toluène comme solvant.

6. Procédé selon la revendication 1, dans lequel, dans l'étape c) du procédé, on utilise comme réactif pour le clivage par réduction de la liaison sulfonimidoylalkyle dans les composés de formule générale Xa l'iodure de samarium-II.

7. Procédé selon la revendication 1, dans lequel, dans les composés de formules générales, Ia', Ia, Ib, II, IX et Xa, R⁴ à chaque fois ne représente pas un hydrogène.

8. Procédé selon la revendication 1, dans lequel on utilise comme groupe protecteur de silyle R¹¹⁰¹ le tert.-butyldiméthylsilyle ou le triméthylsilyle.

9. Procédé selon la revendication 1 pour préparer des composés de formule générale Ia', dans lequel R⁸ représente un hydrogène, un alkyle inférieur, un phényle, un phényl-alkyle inférieur ou un alcoxy inférieur-alkyle inférieur, où R⁶ et R⁷ forment ensemble une liaison et R⁵ et R⁸, avec l'atome de carbone auquel ils sont liés, forment un système cyclique en C₆ aromatique ou dans lequel R⁸ forme avec R⁹ une chaîne alkylène en C₃-C₄.

10. Composés de formule générale Xa selon la revendication 1, où le substituant contenant du soufre en position 5 de l'ossature de base cyclique et le groupe hydroxy en position 3 de l'ossature de base cyclique sont à chaque fois l'un par rapport à l'autre en position trans et où le substituant R⁴ en position 4 et le groupe hydroxy en position 3 de l'ossature de base cyclique sont à chaque fois en position cis l'un par rapport à l'autre.

11. Composés de formule générale Xa selon la revendication 10, où R⁹⁰¹ représente un hydrogène ou forme ensemble avec R⁸⁰¹ une chaîne alkylène en C₃-C₄.

12. Utilisation d'iodure de samarium-(II) pour la désulfuration réductrice de composés d'alkylsulfonimidoyle de formule générale Xa de la revendication 1.

13. Utilisation de (Rₛ)4(S)-isopropyl-2-p-toluoyl-4,5-dihydro[1,2λ⁶, 3]oxathiazol-2-oxyde, (Sₛ)-4(S)-isopropyl-2-p-toluoyl-4,5-dihydro[1,2λ⁶, 3]oxathiazol-2-oxyde, (Rₛ)-4(R)-isopropyl-2-p-toluoyl-4,5-dihydro[1,2λ⁶, 3]oxathiazol-2-oxyde, et de (Sₛ)-4(R)-isopropyl-2-p-toluoyl-4,5-dihydro[1,2λ⁶, 3]oxathizaol-2-oxyde dans le procédé de préparation stéréochimiquement contrôlée de composés azacycliques selon la revendication 1.

14. Utilisation de [Sₛ,N(1S)]-N-[[(tert.-butyldiméthylsilyl)oxy]méthyl]-2-méthylpropyl]-S-méthyl-S-(4-méthylphényl)sulfoximide et de [Rₛ,N(1R)]-N-[1-[[tert.-butyldiméthylsilyl)oxy]méthyl]-2-méthylpropyl]-S-méthyl-S-(4-méthyl-phényl)sulfoximide dans un procédé de préparation stéréochimiquement contrôlée de composés azacycliques selon la revendication 1.

15. Procédé de préparation de composés de formule générale Ib pour désulfuration réductrice de composés de formule générale Xa selon la revendication 1, **caractérisé en ce que** l'on fait réagir un composé de formule Xa dans un solvant polaire aprotique ou dans un mélange de tels solvants à des températures comprises entre -20°C et la température ambiante, et en présence de 2 à 5 équivalents d'une source de protons, sur la base d'un équivalent de la quantité de soufre contenue dans la quantité utilisée de composé de formule Xa, avec du iodure de samarium II selon un rapport molaire compris entre 3:1 et 7:1, sur la base du composé de formule Xa utilisé.
